(19) Europäisches Patentamt
European Patent Office
Office européen des brevets

(11) Publication number: 0 219 308 B1

(12) # EUROPEAN PATENT SPECIFICATION

(45) Date of publication of patent specification: 23.10.91

(51) Int. Cl.[5]: C07D 215/14, C07D 215/12, C07D 215/18, C07D 405/10, A61K 31/47, //(C07D405/10, 307:00,215:00)

(21) Application number: 86307785.5

(22) Date of filing: 08.10.86

The file contains technical information submitted after the application was filed and not included in this specification

(54) **2-Substituted quinolines.**

(30) Priority: 16.10.85 US 787942

(43) Date of publication of application: 22.04.87 Bulletin 87/17

(45) Publication of the grant of the patent: 23.10.91 Bulletin 91/43

(84) Designated Contracting States: AT BE CH DE FR GB IT LI LU NL SE

(56) References cited:
EP-A- 0 110 405
EP-A- 0 181 568
EP-A- 0 200 101
FR-A- 2 445 319
FR-A- 2 578 540
GB-A- 321 738
GB-A- 2 002 764
JP-A-61 246 164
US-A- 4 325 959
US-A- 4 464 533

CHEMICAL ABSTRACTS, vol. 72, no. 17, 27th April 1970, page 369, abstract no. 90223p, Columbus, Ohio, US; M.P. MERTES et al.: "Cofactor inhibitors of thymidylate synthetase. Piperidine and tetrahydroquinoline analogs of tetrahydrofolic acid", & J. MED. CHEM. 1970, 13(2), 276(9)

(73) Proprietor: MERCK FROSST CANADA INC.
16711 Trans-Canada Highway
Kirkland Quebec(CA)

(72) Inventor: Young, Robert N.
216 Senneville
Quebec H9X 3L2(CA)
Inventor: Zamboni, Robert
233 Boul. Jacques Cartier Est
Longueuil Quebec J4L 1E1(CA)
Inventor: Leger, Serge
51 Lamarche
Dollard des Ormeaux(CA)

(74) Representative: Crampton, Keith John Allen et al
D. YOUNG & CO. 10 Staple Inn
London WC1V 7RD(GB)

EP 0 219 308 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 101, no. 25, 17th December 1984, page 751, abstract no. 230319u, Columbus, Ohio, US; G. KARLIVANS et al.: "New method for the synthesis of pyridyl- and quinolylcarbonylarylcarboxylic acids", & KHIM. GETEROTSIKL. SOEDIN. 1984, (9), 1231-4

CHEMICAL ABSTRACTS, vol. 83, no. 1, 7th July 1975, page 807, abstract no. 9726n, Columbus, Ohio, US; T. ENDO et al.: "Reactions of aromatic N-oxides with O-benzoyl aromatic aldehyde cyanohydrins in acetic anhydride", & HETEROCYCLES 1975, 3(1), 19-23

JUSTUS LIEBIGS "Annalen der Chemie", vol. 734, 1970, pages 13-22, Verlag Chemie GmbH, Weinheim/Bergstr., DE; W. RIED et al.: "Darstellung Fluoreszenz-aktiver Nitrile und Imidsäureester"

CHEMICAL ABSTRACTS, vol. 106, no. 15, 13th April 1987, page 627, abstract no. 119700a, Columbus, Ohio, US; & JP-A-61 246 164 (M. HASEGAWA et al.) 01-11-1986

## Description

This invention relates to compounds that act as antagonists of the leukotrienes and as inhibitors of their biosynthesis, and to their preparation and use, inter alia in pharmaceutical formulations.

The leukotrienes and their biological activities, especially their roles in various disease states and conditions have been described (see, for example, European Patent Specification EP-A-0 140 684. Several classes of compounds antagonize the action of leukotrienes in mammals, especially humans. See for example: United Kingdom Patent Specifications GB-A-2 058 785 and GB-A-2 094 301; and European Patent Specification EP-A-0 056 172, EP-A-0 061 800 and EP-A-0 068 739.

EP-A-0 110 405 describes anti-inflammatory and anti-allergic substituted benzenes which are disclosed to be leukotriene inhibitors, i.e., inhibitors of the 5-lipoxygenase pathway.

Various 2-substituted quinolines in which the 2-substituent is a phenyl ring attached by way of a chain or a radical containing an oxygen, nitrogen or sulphur atom, and in which the quinoline and/or phenyl ring may contain other substituents, are disclosed in Chem. Abs. 72(1970), No. 17 p. 369, 90223p; 101 (1984), No. 25 p. 751, 230319u; and 83(1975), No. 1 p. 807, 9726n; in Liebig's "Annalen der Chemie", 734(1970), pages 13 to 22; in US Patent Specifications US-A-4 325 959 and US-A-4 464 533; in European Patent Specifications EP-A-0 181 568 and EP-A-0 200 101; and in UK Patent Specification GB-A-3 217 738.

The compounds of this invention have Formula Ia:

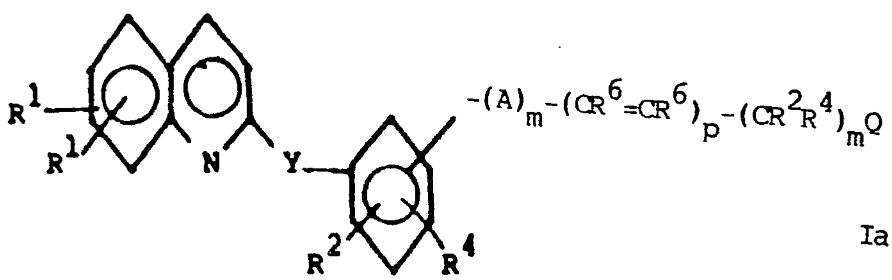

Ia

in which

| | |
|---|---|
| Y is | $-(CR^2=CR^2)_n-$ or $-(C\equiv C)_n-$; |
| $R^1$ is | H, halogen, $C_{1-8}$ alkyl, $C_{2-8}$ alkenyl, $C_{2-8}$ alkynyl, $-CF_3$, $-OR^2$, $-SR^2$, $-SOR^2$, $-SO_2R^2$, $-NR^2R^2$, -CHO, $-COOR^2$, $-(C=O)R^2$, $-C(OH)R^2R^2$, -CN, $-NO_2$, substituted or unsubstituted phenyl, substituted or unsubstituted benzyl, or substituted or unsubstituted phenethyl; |
| $R^2$ is | H, $C_{1-8}$ alkyl, $C_{2-8}$ alkenyl, $C_{2-8}$ alkynyl, $-CF_3$, substituted or unsubstituted phenyl, substituted or unsubstituted benzyl, or substituted or unsubstituted phenethyl; |
| $R^4$ is | H, halogen, $-NO_2$, -CN, $-OR^2$, $-SR_2$, $-NR^2R^2$ or $C_{1-8}$ alkyl; |
| m is | 0 or an integer not exceeding 8; |
| n is | 1 or 2; |
| p is | 0 or 1; |
| A is | $-CR^4R^4-$ or $=C=O$; |
| Q is | $-COOR^2$, tetrazole, |

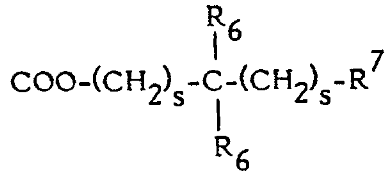

(where s is 0 or an integer not exceeding 3, $R^6$ is hydrogen or $C_{1-4}$ alkyl, and $R^7$ is $OR^8$, $SR^8$ or $NHR^8$, where $R^8$ is a $C_{1-21}$ hydrocarbon radical or a $C_{1-21}$ acyl radical of an organic acyclic or monocyclic carboxylic acid containing not more than one hetero atom in the ring, or $R^7$ is a monocyclic or bicyclic heterocyclic radical containing 3 to 12 nuclear carbons and 1 or 2 N, S and/or 0 hetero atoms, each ring having 5 or 6 atoms), $-CONHSO_2R^{11}$, $-CONR^{10}R^{10}$, or (but only when the sum of m, m and p is greater than 0) $-NHSO_2R^{11}$; or if Q is COOH and

$R^3$ contains an $R^4$ that is -OH, then Q and $R^4$ and the carbons through which they are attached may form a lactone ring with loss of water;

$R^{10}$ is H, $C_{1-6}$ alkyl, $-(C=O)R^{11}$, unsubstituted phenyl or unsubstituted benzyl; and

$R^{11}$ is H, $C_{1-8}$ alkyl, $C_{2-8}$ alkenyl, $C_{2-8}$ alkynyl, $-CF_3$, or unsubstituted phenyl, benzyl, or phenethyl;

substituted phenyl, benzyl or phenethyl means phenyl, benzyl or phenethyl having one or two substituents on the benzene ring selected from $C_{1-6}$ alkyl, $NO_2$, $SCF_3$, halogen, $COR^9$, CN, and $CF_3$, where $R^9$ is $-OR^{10}$, $-SR^{10}$, or $-NR^{10}R^{10}$;

provided that where two or more of any variable are present, each is the same as or different from the other(s);

or are pharmaceutically acceptable salts thereof.

Alkyl, alkenyl, and alkynyl include linear, branched, and cyclic structures. Thus, alkyl would include, for example *n*-butyl, *sec*-butyl, *tert*-butyl and *cyclo*butyl.

Halogen includes F, Cl, Br and I.

It is intended that the definitions of any variable (e.g., $R^1$, $R^2$ or *m*) in a particular molecule is independent of its definitions elsewhere in the molecule. Thus, $-NR^2R^2$ represents *inter alia* $-NH_2$, $-NHCH_3$ and $-NHC_6H_5$.

Some of the compounds of the present invention contain one or more centres of asymmetry and may thus give rise to diastereoisomers and optical isomers. The present invention comprehends such possible diastereoisomers as well as their racemic and resolved optically active forms.

Some of the compounds described herein contain olefinic double bonds and, unless specified otherwise, include both E and Z geometric isomers.

The groups of preferred compounds of Formula Ia are represented by Formulae Ib and Ic:

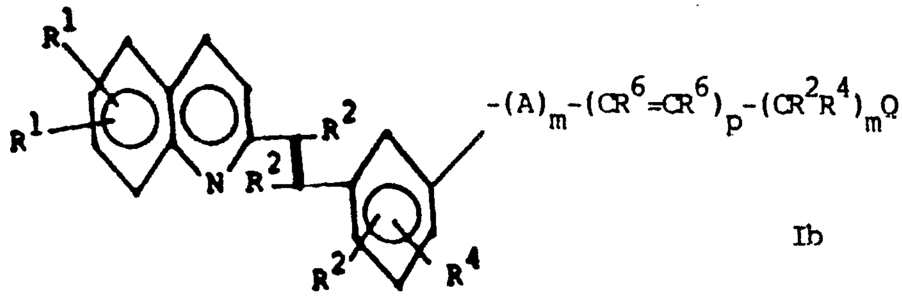

Ib

and

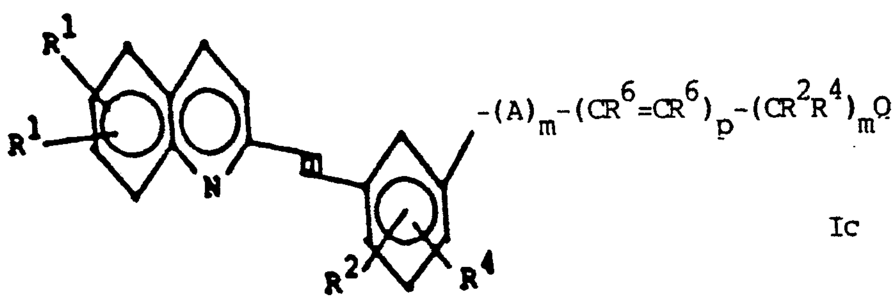

Ic

in which $R^1$ is H, halogen, $CH_3$, $-CF_3$, or $SCF_3$; $R^2$ is H, $C_{1-3}$ alkyl, $C_{2-3}$ alkenyl, or $-CF_3$; and $R^4$, $R^6$, m, p, A and Q are as defined for Formula Ia; and their pharmaceutically acceptable salts.

Preferred compounds of Formulae Ib or IC are those in which Q is $COOR^2$, tetrazole or $COOR^5$, with the remaining substituents being as defined for Ib and Ic respectively.

The pharmaceutically acceptable salts are those prepared from pharmaceutically acceptable non-toxic acids including inorganic acids and organic acids. Such acids include acetic, benzenesulphonic, benzoic, camphorsulphonic, citric, ethanesulphonic, fumaric, gluconic, glutamic, hydrobromic, hydrochloric, isethionic, lactic, maleic, malic, mandelic, methanesulphonic, mucic, nitric, panoic, pantothenic, phosphoric, succinic, sulphuric, tartaric and *p*-toluenesulphonic acids. Particularly preferred are hydrobromic, hydrochloric, phosphoric and sulphuric acids.

Salts derived from inorganic bases include sodium, potassium, lithium, ammonium, calcium, magnesium, ferrous, zinc, copper, manganous, aluminium, ferric and manganic salts, ammonium, potassium, sodium, calcium and magnesium salts being particularly preferred. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, trimethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

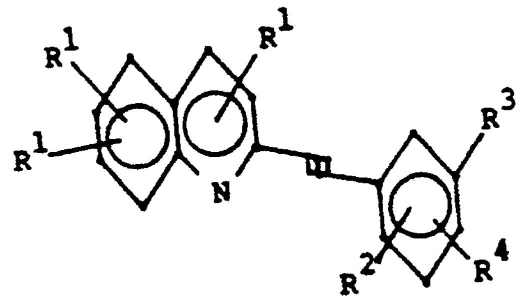

Ic

in which $R^1$ is H, halogen, $CH_3$, $-CF_3$, or $SCF_3$; $R^2$ is H, $C_{1-3}$ alkyl, $C_{2-3}$ alkenyl, or $-CF_3$; p is 0; and $R^3$ to $R^{11}$, m, s, a, Q, W, and X are as defined for Formula I; and their pharmaceutically acceptable salts.

Preferred compounds of Formulae Ib or IC are those in which Q is $COOR^2$, tetrazole or $COOR^5$, with the remaining substituents being as defined for Ib and Ic respectively.

Other compounds within the scope of this invention are best represented by Formula Id:

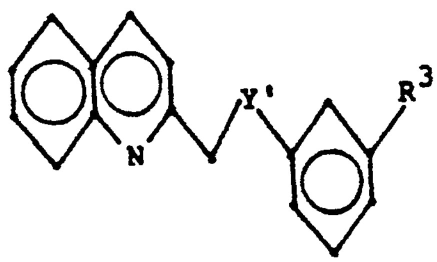

Id

and

for example,

| | Y' | $R^3$ |
|---|---|---|
| 1 | S | COOH |
| 2 | O | COOH |
| 3 | S | $-CH_2-CH_2-COOH$ |
| 4 | O | $-CH_2-CH_2-COOH$ |
| 5 | S | $-CH=CH-COOH$ |
| 6 | O | $-CH=CH-COOH$ |
| 7 | S | $-CHOH-CH(CH_3)-CH_2COOH$ |
| 8 | O | $-CHOH-CH(CH_3)-CH_2COOH$ |
| 9 | S | $-CO-(CH_2)_4-COOH$ |
| 10 | O | $-CO-(CH_2)_4-COOH$ |
| 11 | S | $-CHOH-(CH_2)_4-COOH$ |
| 12 | O | $-CHOH-(CH_2)_4-COOH$ |

The pharmaceutically acceptable salts are those prepared from pharmaceutically acceptable non-toxic acids including inorganic acids and organic acids. Such acids includes acetic, benzenesulphonic, benzoic, camphorsulphonic, citric, ethanesulphonic, fumaric, gluconic, glutamic, hydrobromic, hydrochloric, isethionic, lactic, maleic, malic, mandelic, methanesulphonic, mucic, nitric, panoic, pantothenic, phosphoric, succinic, sulphuric, tartaric and p-toluenesulphonic acids. Particularly preferred are hydrobromic, hydrochloric, phosphoric and sulphuric acids.

Salts derived from inorganic bases include sodium, potassium, lithium, ammonium, calcium, magnesium, ferrous, zinc, copper, manganous, aluminium, ferric and manganic salts, ammonium, potassium, sodium, calcium and magnesium salts being particularly preferred. Salts derived from pharmaceutically acceptable organic non-toxic bases include salts of primary, secondary, and tertiary amines, substituted amines including naturally occurring substituted amines, cyclic amines and basic ion-exchange resins, such as isopropylamine, trimethylamine, diethylamine, triethylamine, tripropylamine, ethanolamine, 2-dimethylaminoethanol, 2-diethylaminoethanol, trimethamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

Compounds of Formula I, including their pharmaceutically acceptable salts and lactone, lactam or thiolactam forms, have been found active as antagonists of SRS-A and especially of leukotriene $D_4$. These compounds also have modest inhibitory activity on leukotriene biosynthesis but are primarily of therapeutic interest as antagonists. The activity of the compounds of Formula I can be detected and evaluated by known methods (see, for example, US Patent Specification US-A-4 296 129).

Because of their activity as leukotriene antagonists or inhibitors, compounds of the present invention are useful as anti-asthmatic, anti-allergic, and anti-inflammatory agents and are useful in treating allergic rhinitis and chronic bronchitis and for amelioration of skin diseases such as psoriasis and atopic eczema. These compounds are also useful to antagonize or inhibit the pathologic actions of leukotrienes on the cardiovascular and vascular systems, which may, for example, result in angina. Compounds of the present invention have also been found useful in the treatment of inflammatory and allergic diseases of the eye, including allergic conjunctivitis, and as cytoprotective agents.

Thus, such compounds may also be used to treat or prevent mammalian (especially, human) disease states such as erosive gastritis; erosive esophagitis; inflammatory bowel disease; ethanol-induced haemorrhagic erosions; hepatic ischemic; noxious-agent-induced damage or necrosis of hepatic, pancreatic, renal, or myocardial tissue; liver parenchymal damage caused by hepatoxic agents such as $CCl_4$ and D-galactosamine; ischemic renal failure; disease-induced hepatic damage; bile-salt-induced pancreatic or gastric damage; trauma- or stress-induced cell damage; and glycerol-induced renal failure. Such compounds are also valuable in the prevention and treatment of other such disease states in which the leukotrienes are the causative factor, e.g. obstructive airway diseases such as allergic bronchial asthma.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of irritants such as strong acids, strong bases, ethanol and hypertonic saline solutions.

Two assays can be used to measure Cytoprotective ability. These assays are (A) an ethanol-induced lesion assay and (B) an indomethacin-induced ulcer assay and are described in European Patent Specification EP-A-0 140 684.

The magnitude of a prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range for anti-asthmatic, anti-allergic or anti-inflammatory use and generally, uses other than cytoprotection, lie within the range of from 0.001 mg to 100 mg per kg body weight of a mammal, preferably 0.01 mg to 10 mg per kg, and especially 0.1 to 1 mg per kg, in single or divided doses. On the other hand, it may be necessary to use dosages outside these limits in some cases.

The exact amount of a compound of the Formula I to be used as a cytoprotective agent will depend on, inter alia, whether it is being administered to heal damaged cells or to avoid future damage, on the nature of the damaged cells (e.g., gastrointestinal ulcerations of nephrotic necrosis), and on the nature of the causative agent. An example of the use of a compound of the invention in avoiding future damage would be coadministration with a non-steroidal anti-inflammatory drug that might otherwise cause such damage (for example, indomethacin). For such use, the compound of Formula I is administered from 30 minutes before to 30 minutes after administration of the NSAID. Preferably it is administered before or simultaneously with the NSAID, for example, in a combined dosage form.

The effective daily dosage level for compounds of Formula I inducing cytoprotection in mammals, especially humans, will generally range from 0.1 mg/kg to 100 mg/kg, preferably 1 mg/kg to 100 mg/kg. The dosage may be administered in single or divided individual doses, and by any suitable route of administration, for example, oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient.

For intravenous administration, a suitable dosage range of anti-asthmatic, anti-inflammatory or anti-allergic use is from 0.001 mg to 10 mg (preferably from 0.01 mg to 1 mg) of a compound of Formula I per kg of body weight per day and for cytoprotective use from 0.1 mg to 100 mg (preferably from 1 mg to 100 mg and especially from 1 mg to 10 mg) of a compound of Formula I per kg of body weight per day.

In the case of an oral composition, a suitable dosage range for anti-asthmatic, anti-inflammatory or anti-allergic use is, e.g. from 0.01 mg to 100 mg of a compound of Formula I per kg of body weight per day, preferably from 0.1 mg to 10 mg per kg, and for cytoprotective use is from 0.1 mg to 100 mg (preferably from 1 mg to 100 mg and especially from 10 mg to 100 mg) of a compound of Formula I per kg of body weight per day.

For administration by inhalation, the compounds of the present invention are conveniently delivered in the form of an aerosol spray from pressurized packs or a nebuliser, or as a powder which may be formulated as a cartridge from which the powder composition may be inhaled with the acid of a suitable device. The preferred delivery system for inhalation is a metered dose inhalation (MDI) aerosol, which may be formulated as a suspension or solution in fluorocarbon propellants.

Suitable topical formulations of Compound I include transdermal devices, aerosols, creams, ointments, lotions and dusting powders.

For the treatment of diseases of the eye, ophthalmic preparations for ocular administration comprising 0.001-1% by weight solutions or suspensions of the compounds of Formula I in an acceptable ophthalmic formulation may be used.

In practical use, the compounds of Formula I can be combined as the active ingredient in intimate admixture with a pharmaceutical carrier and optionally other therapeutic ingredients according to conventional pharmaceutical compounding techniques. The carrier may take a wide variety of forms depending on the form of preparation desired for administration, e.g., oral or parenteral (including intravenous). In preparing the compositions for oral dosage form, any pharmaceutical media may be used, for example, water, glycols, oils, alcohols, flavoring agents, preservatives and coloring agents in the case of oral liquid preparations, such as suspensions, elixirs and solutions; or carriers such as starches, sugars, microcrystalline cellulose, diluents, granulating agents, lubricants, binders and disintegrating agents in the case of oral

solid preparations such as powders, capsules and tablets, with solid oral preparations being preferre d to liquid preparations. Because of their ease of administration, tablets and capsules represent the most advantageous oral dosage unit form. If desired, tablets may be coated by standard aqueous or non-aqueous techniques.

In addition to the common dosage forms set out above, the compounds of Formula I may also be administered by controlled release means and/or delivery devices such as those described in U.S. Patent Specifications US-A-3 845 770, US-A-3 916 899; US-A-3 536 809; US-A-3 598 123; US-A-3 630 200 and US-A-4 008 719.

Pharmaceutical compositions of the present invention suitable for oral administration may be presented as discrete units such as capsules, cachets or tablets each containing a predetermined amount of the active ingredient, as a powder or granules or as a solution of a suspension in an aqueous liquid, a non-aqueous liquid, an oil-in-water emulsion of a water-in-oil liquid emulsion. Such compositions may be prepared by any of the methods of pharmacy but all methods include the step of bringing into association the active ingredient with the carrier which is constituted of one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surfactant or dispersing agent. Molded tablets may be made by molding in a suitable machine, a mixture of the powdered compound moistened with an inert liquid diluent. Desirably, each tablet contains from 2.5 mg to 500 mg of the active ingredient and each cachet or capsule contains from 2.5 to 500 mg of the active ingredient.

The following are examples of representative pharmaceutical dosage forms for the compounds of Formula I:

| Injectable Suspension (I.M.) | mg/ml |
|---|---|
| Compound of Formula I | 10 |
| Methylcellulose | 5.0 |
| Polysorbate 80 | 0.5 |
| Benzyl alcohol | 9.0 |
| Benzalkonium chloride | 1.0 |
| Water for injection to a total volume of 1 ml. | |

| Tablet | mg/tablet |
|---|---|
| Compound of Formula I | 25 |
| Microcrystalline Cellulose | 415 |
| Povidone | 14.0 |
| Pregelatinized Starch | 43.5 |
| Magnesium Stearate | 2.5 |
| | 500 |

| Capsule | mg/capsule |
|---|---|
| Compound of Formula I | 25 |
| Lactose Powder | 573.5 |
| Magnesium Stearate | 1.5 |
| | 600 |

In addition to the compounds of Formula I, the pharmaceutical compositions of the present invention can also contain other active ingredients, such as cyclooxygenase inhibitors, non-steroidal anti-inflammatory drugs (NSAIDs), peripheral analgesic agents such as zomepirac and diflunisal. The weight ratio of the compound of the Formula I to the second active ingredient may be varied and will depend upon the effective dose of each ingredient. Generally, an effective dose of each will be used. Thus, for example, when a compound of Formula I is combined with an NSAID the weight ratio of the compound of Formula I to the NSAID will generally range from 1000:1 to 1:1000. Combinations of a compound of Formula I and other active ingredients will generally also be within the aforementioned range, but in each case, an effective dose of each active ingredient should be used.

NSAIDs can be characterized into five groups, viz. (1) propionic acid derivatives; (2) acetic acid derivatives; (3) fenamic acid derivatives; (4) biphenylcarboxylic acid derivatives; and (5) oxicams, including their pharmaceutically acceptable salts. NSAIDs that can be used in accordance with this invention are those disclosed in European Patent Specification EP-A-0 140 684.

Pharmaceutical compositions comprising the Formula I compounds may also contain inhibitors of the biosynthesis of the leukotrienes such as are disclosed in European Patent Specifications EP-A-0 138 481, EP-A-0 115 394, EP-A-0 136 893 and EP-A-0 140 709, and leukotriene antagonists such as those disclosed in EP-A-0 106 565, EP-A-0 104 885, EP-A-0 056 172 EP-A-0 061 800 and UK Patent Specification GB-A-2 058 785.

Pharmaceutical compositions comprising the Formula I compounds may also contain as the second active ingredient prostaglandin antagonists such as those disclosed in EP-A-0 011 067 or thromboxane antagonists such as those disclosed in US Patent Specification US-A-4 237 160. They may also contain

histidine decarboxylase inhibitors such as α-fluoromethyl-histidine, described in US-A-4 325 961. The compounds of the Formula I may also be advantageously combined with and $H_1$ or $H_2$-receptor antagonist, such as benadryl, dramamine, histadyl, phenergan, terfenadine, acetamezole, cimetidine, ranitidine, famotidine, aminothiadiazoles disclosed in EP-A-0 040 696 and those disclosed in US Patent Specifications US-A-4 283 408, US-A-4 362 736, US-A-4 394 508; and pending US Patent Application No. 301 616, filed 14 September 1981. The pharmaceutical compositions may also contain a $K^+/H^+$ ATPase inhibitor such as omeprazole, which is disclosed in US-A-4 255 431. Another useful pharmaceutical composition comprises a compound of Formula I in combination with serotonin antagonists such as methysergide or the serotonin antagonists disclosed in Nature, vol. 316, pages 126-131, 1985.

Compounds of the present invention can be prepared according to the following methods.

METHOD A

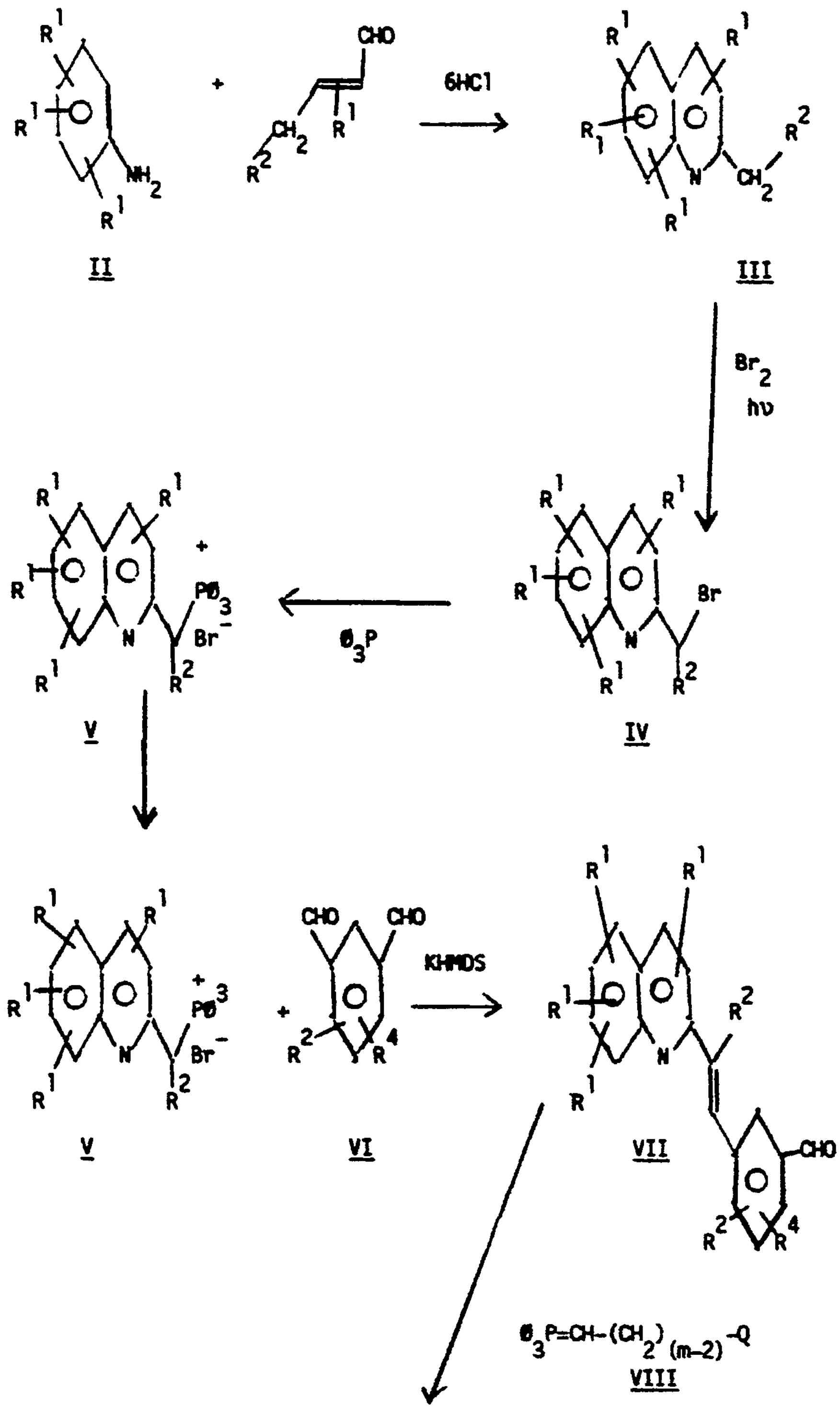


II, III, IV, V, V, VI, VII, VIII

METHOD A (Cont.)

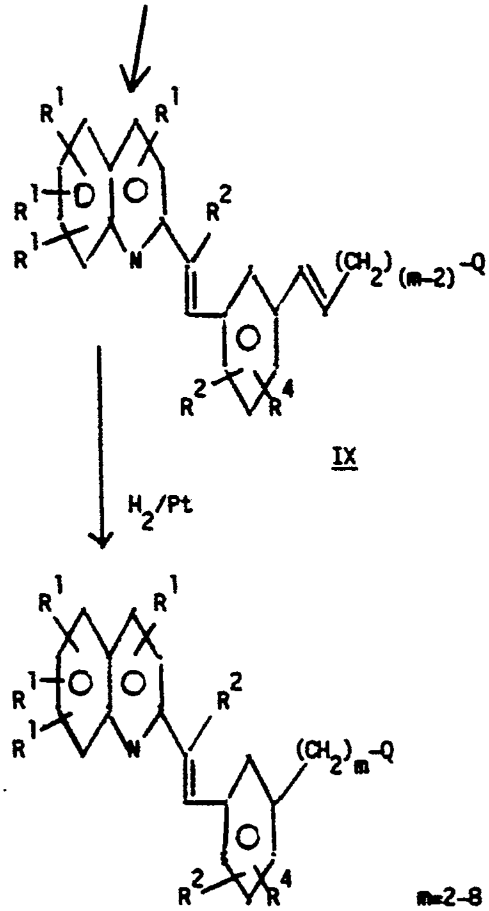


IX

m=2-8

X (I)

METHOD B

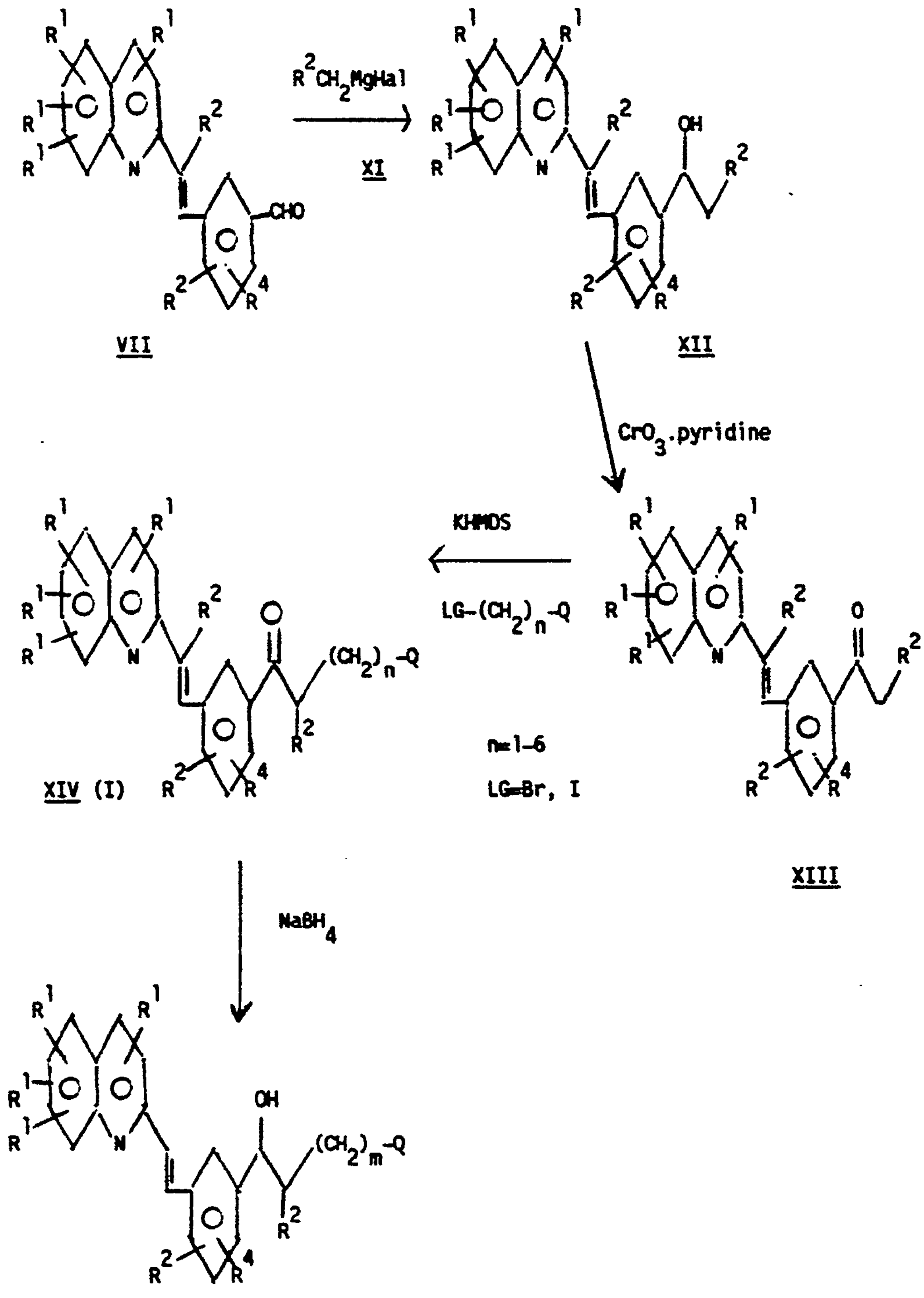


VII

XII

XIII

XIV (I)

XV (I)

METHOD C

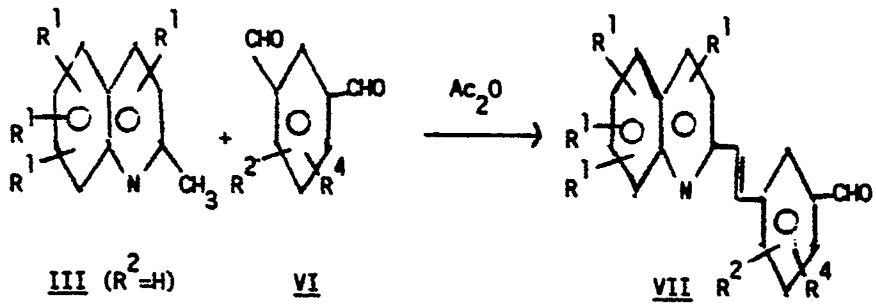


III ($R^2$=H) VI VII

METHOD D

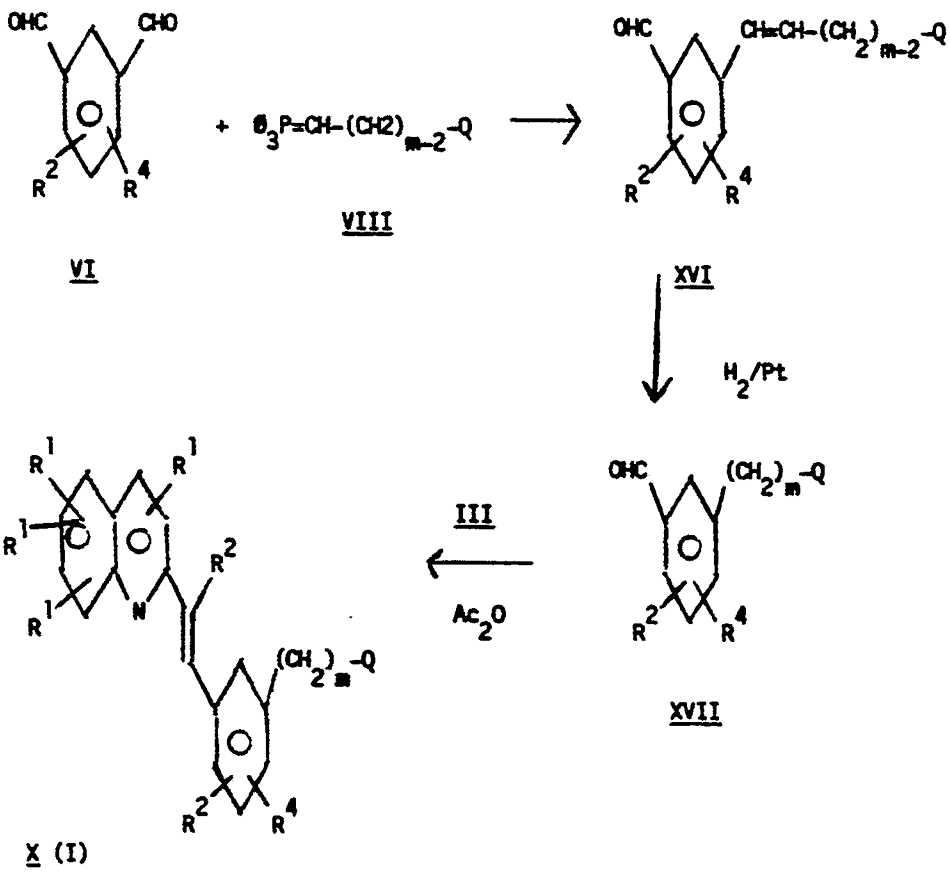


VI VIII XVI XVII X (I)

METHOD E

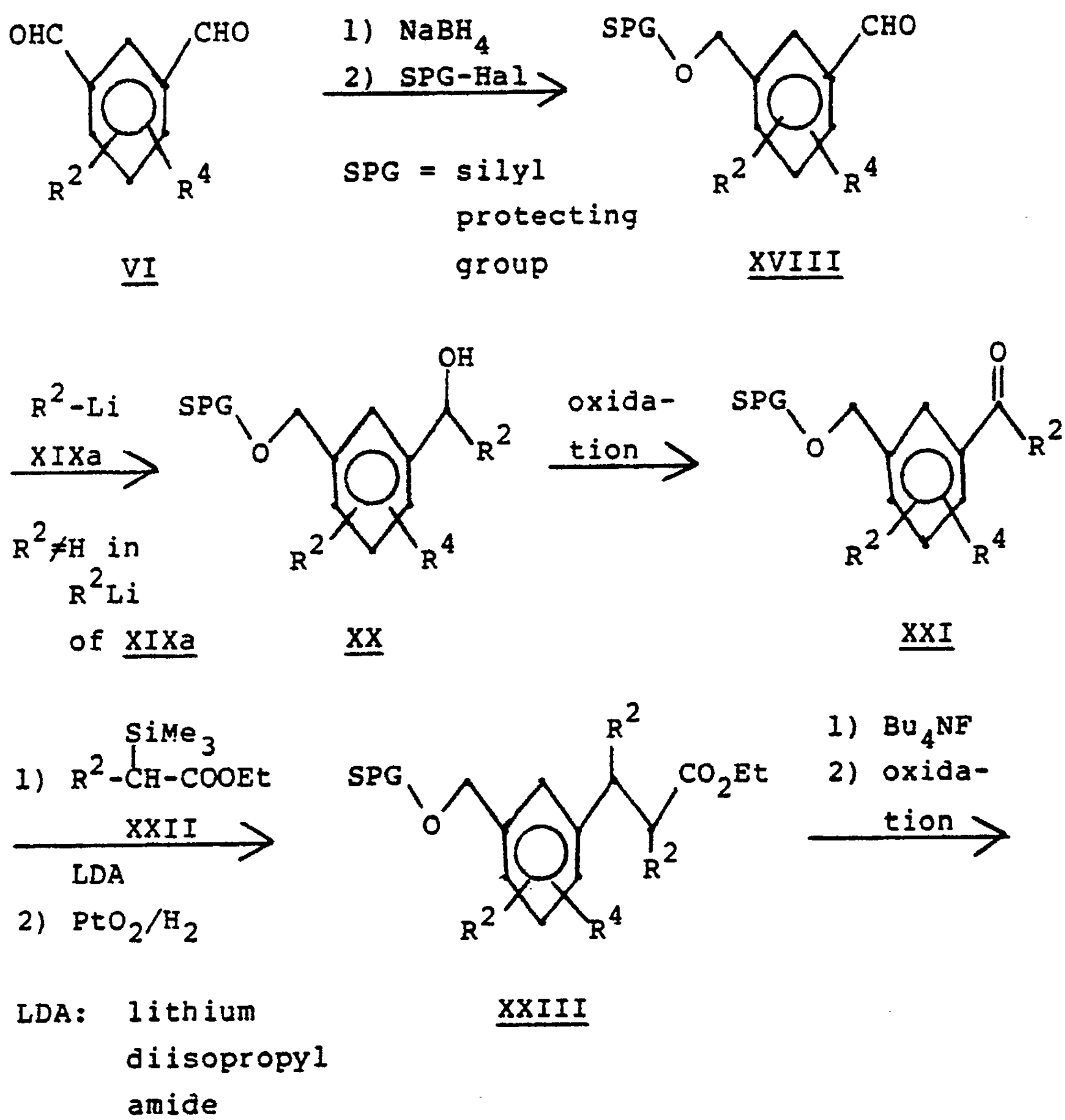
1) $NaBH_4$
2) SPG-Hal
SPG = silyl protecting group
VI
XVIII
$R^2$-Li
XIXa
$R^2 \neq H$ in $R^2Li$ of XIXa
XX
oxida-tion
XXI
1) $R^2$-CH($SiMe_3$)-COOEt
XXII
LDA
2) $PtO_2/H_2$
XXIII
1) $Bu_4NF$
2) oxida-tion
LDA: lithium diisopropyl amide

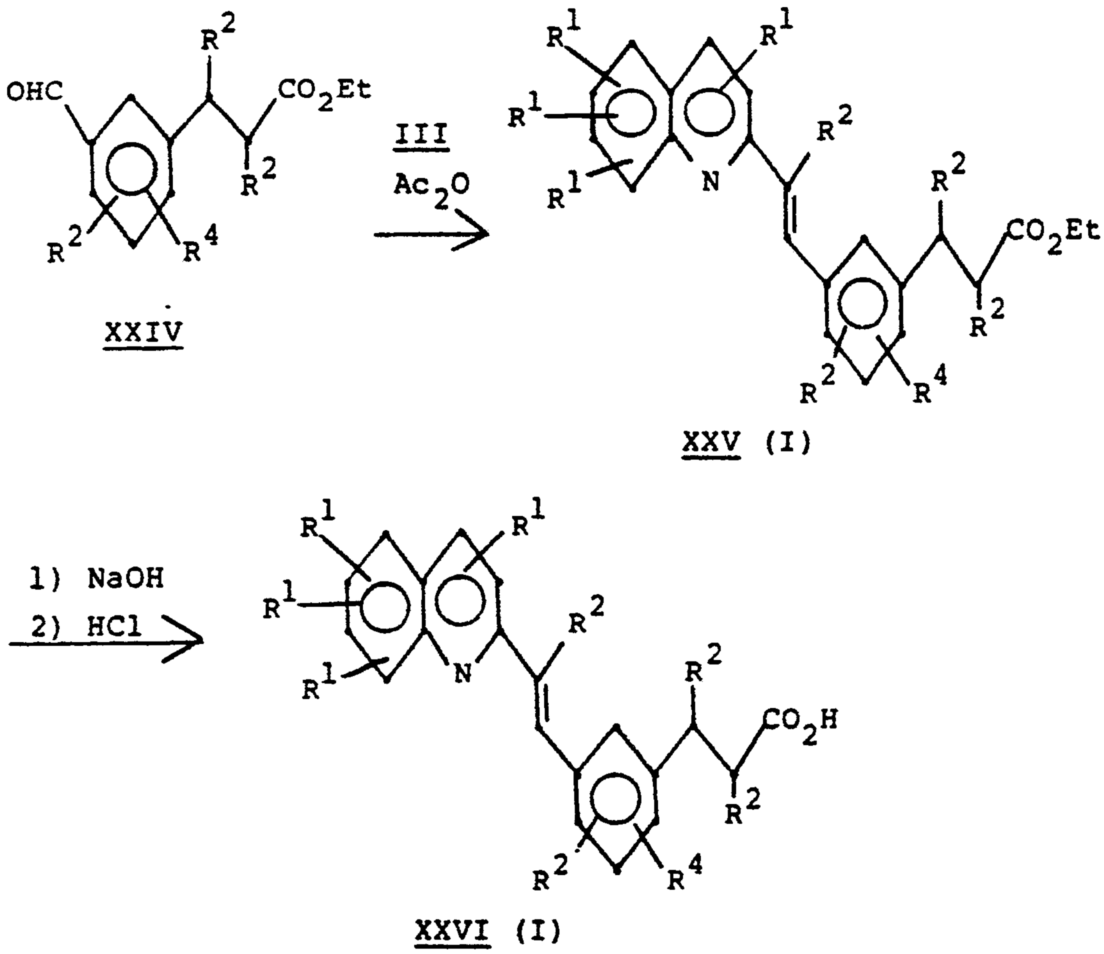


XXIV

XXV (I)

XXVI (I)

METHOD A

Referring to Method A, an aniline derivative of Formula II is reacted by heating with a crotonaldehyde and a strong mineral acid such as aqueous hydrochloric acid to provide the substituted quinaldine derivative of structure III. When II is unsymmetrical two regioisomers of II may be obtained. The products are purified by precipitation of the zinc chloride adducts or by standard chromatographic techniques. Derivative III is treated with a halogen source such as $Br_2$ in the presence of light to afford quinaldine IV. This product is treated with triphenylphosphine in an inert solvent such as toluene with heating to give phosphonium salt V.

Quinaldine derivative of general structure V is reacted with a strong base such as lithium hexamethyldisilazane in an inert solvent at low temperature followed by reaction of the resulting ylid with dialdehyde of general structure VI to provide the vinyl quionoline VII. This product is treated with an ylid VIII to provide adduct IX. The double bond in IX is reduced by treatment with $H_2$ and a suitable catalyst such as $PtO_2$ to provide 2-styryl quionoline derivatives of general structure X. Both compounds IX and X are representatives of Formula I compounds.

Alternatively, VI can be reacted first with ylid VIII. The double bond is then reduced and then the remaining aldehyde is reacted with quinaldine derivative of general structure V to provide X.

METHOD B

According to method B aldehyde VII is reacted with a Grignard reagent XI, or an equivalent organolithium reagent, to provide alcohol XII. Alcohol XII is oxidized using a suitable oxidizing agent such a $CrO_3$.pyridine to provide ketone XIII. Ketone XIII is depronated with a strong base such as potassium hexamethyldisilazane in an inert solvent at low temperature. The resulting enolate is reacted with an alkanoic acid, ester, nitrile or tetrazole terminally substituted with a good leaving group (LG) such as Br or I to provide adducts XIV. The product is reduced with a suitable reducing agent such as $NaBH_4$ to provide quinoline alcohols XV. Both XIV and XV are representative compounds of Formula I.

METHOD C

Referring to Method C, quinoline derivative III can be reacted with a dialdehyde of structure VI by heating with a dehydrating agent, preferably acetic anhydride, to provide the 2-styryl quionoline aldehyde of structure VII. Quinoline VII is then processed as in Methods A and B.

METHOD D

Method D is an alternative synthesis of compounds of Formula X (Method A). In this method dialdehyde VI is condensed with ylid VIII to yield the olefinic aldehyde XVI, which is reduced to aldehyde XVII by treatment with $H_2$ and a catalyst such as $PtO_2$. Quinaldine III and aldehyde XVII are then condensed in a manner similar to that described under Method C to obtain compound X, which is a structure representative of the Formula I compounds.

METHOD E

Method E is useful for preparing benzenepropanoic acid versions of compound I (e.g., XXV and XXVI) bearing a substituent in the α- or the β-position of the propanoic acid. Thus, dialdehyde VI is half-reduced, the alcohol group protected, and the resulting XVIII is reacted with XIXa to yield XX, which is oxidized to XXI by conventional oxidizing agents such as pyridinium chlorochromate (PCC). Compound XXI is condensed with silyl ester XXII and the resulting α,β-unsaturated ester is reduced catalytically to XXIII. The protected alcohol group in XXIII is converted by known methodology to the aldehyde XXIV, which is condensed with III according to Method C to produce XXV. Compound XXV may then be hydrolysed by aqueous base. The salt of the resulting acid may be isolated, or if the free acid is desired, the solution containing the salt may be acidified to yield the acid XXVI. Both XXV and XXVI are representative compounds of Formula I.

The invention is further defined by reference to the following illustrative examples.

All temperatures are in degrees Celsius.

EXAMPLE 1

4-(2-(quinolin-2-yl)ethenyl)benzoic acid methyl ester

Step 1: Preparation of 4-(2-(quinolin-2-yl)ethenyl)benzaldehyde

To a suspension of (quinolin-2-ylmethyl)triphenylphosphonium bromide (10 g) in THF (200 ml) was added dropwise potassium hexamethyl disilazane 0.66 M in toluene) (33 ml). The suspension was stirred 1 hour at -78° C. This solution was added dropwise to a solution of terephthalaldehyde (4 g) in THF. The resulting brown solution was stirred 2 hours at -78°. The reaction mixture was quenched with $NH_4OAc$ buffer, extracted with ethyl acetate, dried over $Na_2SO_4$, filtered and evaporated. Flash chromatography of the residue using 20% to 30% ether in hexane afforded the title compound: m.p. 107-108°.

STEP 2:

A mixture of the aldehyde (0.44 g) from step 1, $MnO_2$ (activated) 2.0 g, acetic acid (0.29 ml) and NaCN (1.00 g) in methanol (100 ml) was stirred 30 minutes and partitioned between ethyl acetate and $H_2O$. The organic layer was washed with $H_2O$, dried on $Na_2SO_4$, filtered, and evaporated. Purification by flash chromatography using 20% ether in hexane afforded the title compound: m.p. 135-136°.

Anal. Calc'd for $C_{19}H_{15}NO_2$

Calc'd: C, 78.9; H, 5.2; N, 4.8.

Found: C, 78.92; H, 5.36; N, 4.79.

EXAMPLE 2

4-(2-(quinolin-2-yl)ethenyl)benzoic acid

To a solution of the methyl ester (Example 1) (47 mg) in THF (3 ml) and MEOH (3 ml) was added 0.2 ml of 3N NaOH. The mixture was stirred at room temperature for 4 days. The solvent were removed *in vacuo* and water was added (15 ml). With vigorous stirring 1:1 water/AcOH was added until the pH of the solution reached 5. The aqueous phase was extracted with ethyl acetate, dried and evaporated to yield the

title product: m.p. 251-252°.
Anal. calc'd for $C_{18}H_{13}NO_2$
Calc'd: C, 78.5; H, 4.8; N, 5.09.
Found: C, 78.14; H, 4.97; N, 4.93.

EXAMPLE 3
3-(2-(quinolin-2-yl)ethenyl)benzenepropanoic acid ethyl ester

STEP 1: Preparation of 3-(3-formylphenyl)propen-2-oic acid ethyl ester

To a solution of isophthaldehyde (4.0 g) in THF (200 ml) at 0° was added carboethoxymethylene-triphenylphosphorane (10.4 g). The reaction mixture was stirred 1.5 hours at room temperature. The mixture was evaporated and purified by flash chromatography using 10 to 25% ether in hexane as solvent to yield the title compound, which was used as such for the next step.

STEP 2: Preparation of 3-formylbenzenepropanoic acid ethyl ester

A solution of the olefin from step 1 (6.02 g) in ethyl acetate (200 ml) and $PtO_2$ (200 mg) was shaken for 6 hours on a Parr hydrogenator at 16 psi of hydrogen. The reaction mixture was filtered and evaporated. Flash chromatography of the residue using 30% ether in hexane afforded the title compound, which was used as such for the next step.

STEP 3:

A solution of aldehyde (Step 2) (1.0 g) acetic anhydride (5.0 ml) and quinaldine (0.66 ml) was heated at 125° for 64 hours. The solvents were removed on a rotary evaporator. Chromatography of the residue using ether in hexane (20 to 30%) afforded an oil. Crystallization from ether/hexane afforded the title compound: m.p. 72-73°.
Anal. calc'd for $C_{22}H_{21}NO_2$
Calc'd: C, 79.74; H, 6.39; N, 4.23.
Found: C, 80.05; H, 6.53; N, 4.22.

EXAMPLE 4
3-(2-(Quinolin-2-yl)ethenyl)benzenepropanoic acid

To a solution of the title ester of Example 3 (470 mg) dissolved in THF (15 ml) and MEOH (6 ml) was added 1N sodium hydroxide (2 ml). After stirring 1 hour at room temperature the reaction mixture was acidified to pH 3, diluted with water, and extracted with ethyl acetate. The organic phase was dried and evaporated to give the title compound: m.p. 139-141°.

EXAMPLE 5
3-(2-(quinolin-2-yl)ethenyl)-γ-oxo-β-methylbenzenebutanoic acid methyl ester

STEP 1: Preparation of 3-(2-(quinolin-2-yl)ethenyl)benzaldehyde

Using the procedure described in Example 1 (Step 1) but starting with isophthalaldehyde in place of terephthalaldehyde the title compound was obtained: m.p. 88-89°.

STEP 2: Preparation of 2-(2-(3-(1-hydroxypropyl)phenyl)ethenyl)quinoline

To a solution of the aldehyde (2.4 g) (Step 1) in THF (80 ml) was added 2.9 M ethyl magnesium bromide (5.8 ml) dropwise. The reaction was stirred 40 minutes at -78°, quenched with $NH_4OAc$ (aq.), and extracted with ethylacetate. The organic layer was dried and evaporated. Flash chromatography of the residue using 20 to 30% ether in hexane afforded the title compound: m.p. 125-126°.

STEP 3: Preparation of 1-(3-(2-(quinolin-2-yl)ethenyl)phenyl)propanone

To a solution of alcohol (1.8 g) (Step 2) in ethyl acetate (100 ml) was added in 3 portions over 5 days

6.4 g of $MnO_2$. The reaction mixture was filtered on a Celite (diatomaceous earth) plug, and evaporated. Chromatography of the residue using 25 to 35% ether in hexane yielded the title ketone which was used as such in the next step.

STEP 4:

To a solution of potassium hexamethyl disilizane (7.7 ml, 0.63 M) in THF (6 ml) at -78° was added dropwise a solution of the ketone (Step 3) (1.3 g) in THF (25 ml). The reaction mixture was stirred 45 min at -78°. Methyl bromoacetate (0.5 ml) was added dropwise and the reaction mixture was stirred 1.5 hours at -78°. The reaction was quenched with $NH_4OAc$ buffer (25% aq. sol.), extracted with ethyl acetate, dried ($Na_2SO_4$) and evaporated. Chromatography of the residue using 25 to 40% ether in hexane afforded the title compound. Mass spectrum showed a molecular ion at m/e 359.
Anal. Calc'd for $C_{23}H_{21}NO_3$
Calc'd: C, 76.87; H, 5.89; N, 3.90.
Found: C, 76.93; H, 6.12; N. 3.92.

EXAMPLE 6
3-(2-(quinolin-2-yl)ethenyl)-γ-hydroxy-β-methylbenzenebutanoic acid methyl ester

To a solution of the title ketone of Example 5 (0.9 g) and $CeCl_3$ (5 mg) in MeOH (18 ml) at -20° was added $NaBH_4$ (47 mg). The reaction mixture was stirred 3 hours at -20° and quenched with $NH_4OAc$ buffer. The solution was extracted with ethyl acetate, dried ($Na_2SO_4$) and evaporated. Flash chromatography of the residue using 50% ether in hexane yielded the title compound, which was used as is in the next Example.

EXAMPLE 7
[βR,γS and βS,γR], and [βS,γS and βR,γR]-3-(2-quinolin-2-yl)-ethenyl)-γ-hydroxy-β-methylbenzenebutanoic acid lactone

A solution of the hydroxy ester of Example 6 (0.64 g) and $K_2CO_3$ (0.4 g) in methyl ethyl ketone (25 ml) was refluxed for 4 hours. The reaction mixture was filtered and evaporated. Flash chromatography of the residue using 70% ether in hexane afforded the [βS,γS and βR,γR] isomer and the [βR,γS and βS,γR] isomer of the title compound.
[βS,γS and βR,γR] isomer
P.m.r. ($CDCl_3$) δ 1.24 (d, 3H), 2.38 (dd, 1H), 2.52 (m, 1H), 2.84 (dd, 1H), 5.00 (d, 1H), 7.30 (d, 1H), 7.40 to 7.56 (m, 4H), 7.62 to 7.77 (m, 4H), 7.81 (d, 1H), 8.08 (d, 1H), 8.17 (d, 1H).
[βR,γS and βS,γR] isomer
P.m.r. ($CDCl_3$) δ 0.75 (d, 1H), 2.40 (d, 1H), 2.9 (m, 2H), 5.65 (d, 1H), 7.21 (d, 1H), 7.40 to 7.56 (m, 4H), 7.62 (bd, 1H), 7.66 to 7.77 (m, 3H), 7.81 (d, 1H), 8.08 (d, 1H), 8.17 (d, 1H).

EXAMPLE 8
[βR,γS and βS,γR]-3-(2-(Quinolin-2-yl)ethenyl)-γ-hydroxy-β-methylbenzenebutanoic acid sodium salt

A solution of the [βR,γS and βS,γR] lactone (275 mg) from Example 7 in THF (9 ml), MeOH (5 ml), 1N NaOH (1.6 ml) and $H_2O$ (ml) was stirred overnight. The organic solvents were evaporated. The residue was applied to an XAD-8 chromatography column. Elution with $H_2O$ removed all the salts. Elution with ethanol afforded the title compound.
Anal. calc'd for $C_{22}H_{20}NNaO_3.1/2H_2O$
Calc'd: C, 69.83; H, 5.54; N, 3.70.
Found: C, 70.35; H, 5.64; N, 3.54.

EXAMPLE 9
[βS,γS and βR,γR]-3-(2-quinolin-2-yl)ethenyl)-γ-hydroxy-β-methylbenzenebutanoic acid sodium salt

A solution of the [βS,γS and βR,γR] lactone (147 mg) from Example 7 in THF (5 ml), MeOH (2.5 ml), 1N NaOH (0.9 ml) and $H_2O$ (1.6 ml) was stirred overnight. The organic solvents were removed under vacuo. The residue was applied to a chromatography column of XAD-8. Elution with water removed the salt. Elution with ethanol afforded the title compound.
Anal. calc'd for $C_{22}H_{20}NNaO_3.1/2H_2O$

Calc'd: C, 69.83; H, 5.54; N, 3.70; Na, 6.08.
Found: C, 70.39; H, 5.46; N, 3.67; Na, 5.85.

EXAMPLE 10

3-(2-(7-Chloroquinolin-2-yl)ethenyl)-β-methylbenzenepropanoic acid

STEP 1: Preparation of 3-hydroxymethylbenzaldehyde

Sodium borohydride was added in portions (50 mg) over 4 hours to a solution of isophthaldehyde (4.8 g) in ethanol (50 ml) until 20% of the diol was formed by t.l.c. The reaction mixture was quenched with aqueous $NH_4OAc$, extracted with ethyl acetate, dried, and evaporated. Purification by flash chromatography using 50% ether in hexane afforded the title compound.
P.m.r. ($CD_3COCD_3$) δ 4.5 (t, 1H), 4.75 (d, 2H), 7.55 (t, 2H), 7.70 (m, 1H), 7.80 (m, 1H), 7.95 (m, 1H), 10.0 p.p.m. (s, 1H).

STEP 2: Preparation of 3-(diphenyl t-butylsilyloxymethyl)benzaldehyde

To a solution of alcohol (Step 1) (3.5 g) in $CH_2Cl_2$ (70 ml) was added triethylamine (4.3 ml), t-butyldiphenylsilylchloride (8.1 ml) and dimethylaminopyridine (ca. 100 mg). The reaction mixture was stirred 4 days at room temperature, quenched with aqueous $NH_4OAc$, extracted with $CHCl_3$, dried, and evaporated. Flash chromatography of the residue afforded the title compound.
P.m.r. ($CD_3COCD_3$) δ 1.0 (s, 9H), 4.9 (s, 2H), 7.3-8.0 (m, 14H), 10.0 p.p.m. (s, 1H).

STEP 3: Preparation of 1-(3-(diphenyl-t-butylsilyloxymethyl)phenyl)ethanol

To a solution of aldehyde (Step 2) (3.7 g) in 70 ml of THF at -78° was added dropwise methyl lithium (10 ml of 1.3M). The reaction mixture was stirred 15 minutes at -78°, quenched with aqueous $NH_4OAc$, extracted with ethyl acetate, dried, and evaporated. Flash chromatography of the residue using 20% ethyl acetate in hexane afforded the title compound.
P.m.r. ($CD_3COCD_3$) δ 1.1 (s, 9H), 1.50 (d, 3H), 4.25 (d, 1H), 4.8-4.90 (m, 3H), 7.3-7.8 p.p.m. (m, 14H).

STEP 4: Preparation of 3-(diphenyl-t-butylsilyloxymethyl)acetophenone

To a suspension of powdered 4A molecular sieves (15 g) and alcohol (Step 3) (2.5 g) in $CH_2Cl_2$ (100 ml) was added pyridinium chlorochromate (8.0 g). The resulting mixture was stirred 40 minutes at room temperature. Ether (400 ml) was added. The suspension was filtered on a silica pad and the filtrate evaporated. Flash chromatography of the residue using 10% ethyl acetate in hexane afforded the title compound.
P.m.r. ($CD_3COCD_3$) δ 1.1 (s, 9H), 2.55 (s, 3H), 4.9 (s, 2H), 7.40-8.0 p.p.m. (m, 14H).

STEP 5: Preparation of ethyl 3-(3-(diphenyl-t-butylsilyloxymethyl)phenyl)-2-butenenoate

To a solution of butyl lithium (3.5 ml of 1.55M) in THF (10 ml) at -78° was added diisopropylamine (0.75 ml). The solution was stirred 1 hour at -78°. Ethyl (trimethylsilyl) acetate (1.0 ml) was added dropwise. After stirring 30 minutes at -78°, the acetophenone (Step 4) (2.08 g) in THF was added dropwise. The solution was stirred 30 minutes at -78° and then warmed to room temperature (3 hours). The reaction mixture was quenched with aqueous $NH_4OAc$, extracted with ethyl acetate, dried, and evaporated. Flash chromatography of the residue using 10% ether in hexane afforded the title compound which was used as is for the next step.

STEP 6: Preparation of ethyl 3-(diphenyl t-butylsilyloxymethyl)-β-methylbenzenepropanoate

The olefin (Step 5) (1.8 g) and platinum oxide (180 mg) in ethyl acetate were stirred 4 hours with hydrogen (1 atm.). The catalyst was filtered and the solution evaporated. Flash chromatography of the residue using 10% ether in hexane afforded the title compound.
P.m.r. ($CD_3COCD_3$) δ 1.1 (s, 9H), 1.1 (t, 3H), 1.35 (d, 3H), 2.55 (d, 2H), 3.20 (m, 1H), 4.0 (q, 2H), 4.80 (s, 2H), 7.1-7.8 p.p.m. (M, 14H).

STEP 7: Preparation of ethyl 3-(hydroxymethyl)-β-methylbenzenepropanoate

To a solution of silyl ether (Step 6) (1.5 g) in THF (2 ml) was added tetrabutylammonium fluoride (5 ml of 1M solution). The reaction mixture was stirred 2 hours at room temperature and evaporated under nitrogen. Flash chromatography of the residue using 50 to 70% ether in hexane afforded the title compound.
P.m.r. ($CD_3COCD_3$) δ 1.1 (t, 3H), 1.25 (d, 3H), 2.55 (d, 2H), 3.25 (m, 1H), 4.0 (q, 2H), 4.30 (t, 1H), 4.60 (d, 2H), 7.1-7.5 p.p.m. (m, 4H).

STEP 8: Preparation of ethyl 3-(formyl)-β-methylbenzenepropanoate

Using the procedure described in Example 10 Step 4 but substituting the alcohol from Step 7 there was obtained the title compound.
P.m.r. ($CD_3COCD_3$) δ 1.1 (t, 3H), 1.3 (d, 3H), 2.65 (d, 2H), 3.35 (m, 1H), 4.0 (q, 2H), 7.5-7.85 (m, 4H), 10.0 p.p.m. (s, 1H).

STEP 9: Preparation of ethyl 3-(2-(7-chloroquinolin-2-yl)ethenyl)-β-methylbenzenepropanoate

A solution of the aldehyde (Step 8) (550 mg) and 7-chloroquinaldine (445 mg) in acetic anhydride (4.0 ml) were heated at 125° for 48 hours. The reaction mixture was evaporated. Flash chromatography of the residue using 3% ethyl acetate in toluene afforded the title compound.
P.m.r. ($CD_3COCD_3$) δ 1.1 (t, 3H), 1.30 (d, 3H), 2.65 (d, 2H), 3.40 (m, 1H), 4.05 (q, 2H), 7.3-7.7 (m, 6H), 7.8-8.05 (m, 4H), 8.35 p.p.m. (d, 1H).

STEP 10:

To a solution of ethyl ester (Step 9) (535 mg) in THF (15 ml), MeOH (6 ml) was added 1N NaOH (5.5 ml). The reaction mixture was stirred 3 hours at room temperature. The reaction mixture was acidified with 2N HCl and $H_2O$ (100 ml) was added. The suspension was extracted with ethyl acetate (2 x 100 ml) dried and evaporated to give the title compound.
P.m.r. ($CD_3COCD_3$): δ 1.3 (d, 3H), 2.5-2.70 (m, 2H), 3.30 (m, 1H), 7.25-7.70 (m, 6H), 7.85-8.03 (m, 4H), 8.35 p.p.m. (d, 1H).

EXAMPLE 11

EXAMPLE 12
3-(2-(7-Bromoquinolin-2-yl)ethenyl)-β-methylbenzenepropanoic acid

Following the procedure of Example 10, but replacing 7-chloroquinaldine by 7-bromoquinaldine, there is obtained the title compound.

EXAMPLE 13
3-(2-(7-Trifluoromethylquinolin-2-yl)ethenyl)-β-methylbenzenepropanoic acid

Following the procedure of Example 10, but replacing 7-chloroquinaldine by 7-trifluoromethylquinaldine, there is obtained the title compound.

EXAMPLE 14
3-(2-(6,7-Dichloroquinolin-7-yl)Ethenyl)-β-methylbenzenepropanoic acid

Following the procedure of Example 10, but replacing 7-chloroquinaldine by 6,7-dichloroquinaldine, there is obtained the title compound.

EXAMPLE 15
[βR,γS and βS,γR]-3-(2-(7-Bromoquinolin-2-yl)ethenyl)-γ-hydroxy-β-methylbenzenebutanoic acid sodium salt

Starting with 7-bromoquinaldine and isophthalaldehyde, and following the procedures of Examples 3-(step 3), 5(steps 2, 3, 4), 6, 7, and 8, there was obtained the title compound.
Anal. calc'd for $C_{22}H_{19}BrNNaO_3.1/2\ H_2O$
Calc'd: C, 57.78; H, 4.41; N, 3.06; Na, 5.03; Br 17.47.
Found: C, 57.75; H, 4.36; N, 2.88; Na, 5.20; Br 18.32.

EXAMPLE 16
[βS,γS and βR,γR]-3-(2-(7-Bromoquinolin-2-yl)ethenyl-γ-hydroxy-β-methylbenzenebutanoic acid sodium salt

Starting with 7-bromoquinaldine and isophthalaldehyde, and following the procedures of Examples 3-(step 3), 5(steps 2, 3, 4), 6, 7, and 9, there was obtained the title compound.
Anal. calc'd for $C_{22}H_{19}BrNNaO_3.1/2\ H_2O$
Calc'd: C, 57.78; H, 4.41; N, 3.06; Na, 5.03.
Found: C, 57.84; H, 4.41; N, 3.09; Na, 4.65.

Ketone derivatives of 2-styryl quinolines used as intermediate compounds are claimed in our case No. 17337, which is being filed at the same time as this application

**Claims**
**Claims for the following Contracting States : BE, CH, DE, FR, GB, IT, LI, LU, NL, SE**

**1.** A compound of the formula Ia:

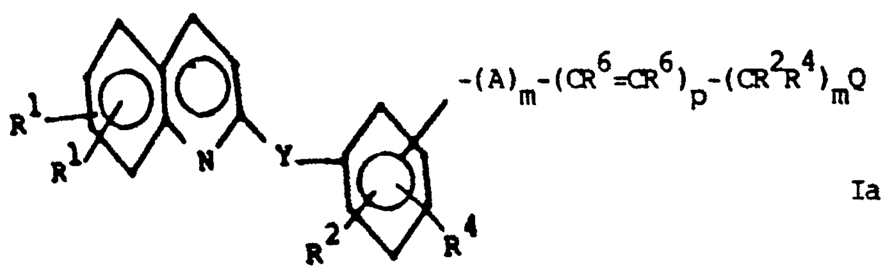

Ia

in which

| | |
|---|---|
| Y is | $-(CR^2=CR^2)_n-$ or $-(C\equiv C)_n-$; |
| $R^1$ is | H, halogen, $C_{1-8}$ alkyl, $C_{2-8}$ alkenyl, $C_{2-8}$ alkynyl, $-CF_3$, $-OR^2$, $-SR^2$, $-SOR^2$, $-SO_2R^2$, $-NR^2R^2$, -CHO, $-COOR^2$, $-(C=O)R^2$, $-C(OH)R^2R^2$, -CN, $-NO_2$, substituted or unsubstituted phenyl, substituted or unsubstituted benzyl, or substituted or unsubstituted phenethyl; |
| $R^2$ is | H, $C_{1-8}$ alkyl, $C_{2-8}$ alkenyl, $C_{2-8}$ alkynyl, $-CF_3$, substituted or unsubstituted phenyl, substituted or unsubstituted benzyl, or substituted or unsubstituted phenethyl; |
| $R^4$ is | H, halogen, $-NO_2$, -CN, $-OR^2$, $SR^2$, $-NR^2R^2$ or $C_{1-8}$ alkyl; |
| m is | 0 or an integer not exceeding 8; |
| n is | 1 or 2; |
| p is | 0 or 1; |
| A is | $-CR^4R^4-$ or $=C=O$; |
| Q is | $-COOR^2$, tetrazole, |

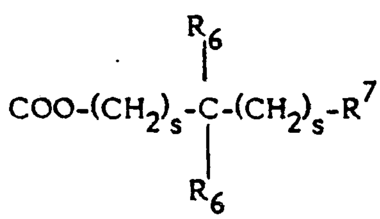

(where s is 0 or an integer not exceeding 3, $R^6$ is hydrogen or $C_{1-4}$ alkyl, and $R^7$ is $OR^8$, $SR^8$ or $NHR^8$, where $R^8$ is a $C_{1-21}$ hydrocarbon radical or a $C_{1-21}$ acyl radical of an organic acyclic or monocyclic carboxylic acid containing not more than one hetero atom in the ring, or $R^7$ is a monocyclic or bicyclic heterocyclic radical containing 3 to 12 nuclear

carbons and 1 or 2 N, S and/or 0 hetero atoms, each ring having 5 or 6 atoms), $-CONHSO_2R^{11}$, $-CONR^{10}R^{10}$, or (but only when the sum of m, n and p is greater than 0) $-NHSO_2R^{11}$; or if Q is COOH and $-(CR^2R^4)_m-$ contains an $R^4$ that is -OH, then Q and $R^4$ and the carbons through which they are attached may form a lactone ring with loss of water;

$R^{10}$ is H, $C_{1-6}$ alkyl, $-(C=O)R^{11}$, unsubstituted phenyl or unsubstituted benzyl; and

$R^{11}$ is H, $C_{1-8}$ alkyl, $C_{2-8}$ alkenyl, $C_{2-8}$ alkynyl, $-CF_3$, or unsubstituted phenyl, benzyl, or phenethyl;

substituted phenyl, benzyl or phenethyl means phenyl, benzyl or phenethyl having one or two substituents on the benzene ring selected from $C_{1-6}$ alkyl, $NO_2$, $SCF_3$, halogen, $COR^9$, CN, and $CF_3$, where $R^9$ is $-OR^{10}$, $-SR^{10}$, or $-NR^{10}R^{10}$ ;

provided that where two or more of any variable are present, each is the same as or different from the other(s);

or are pharmaceutically acceptable salts thereof.

2. A compound of the Formula Ib:

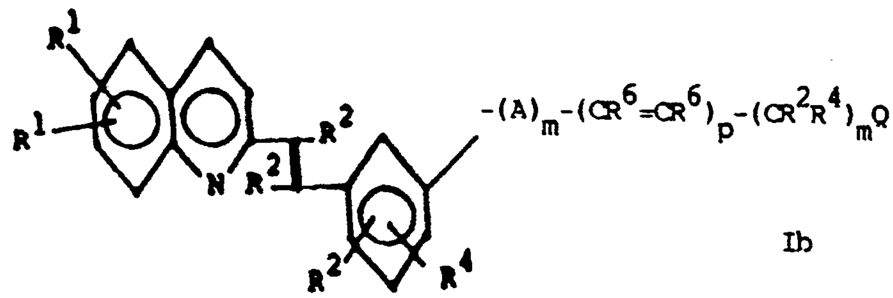

Ib

in which $R^1$ is H, halogen, $CH_3$, $-CF_3$ or $SCF_3$; $R^2$ is H, $C_{1-3}$ alkyl, $C_{2-3}$alkenyl, or $-CF_3$; and $R^4$, $R^6$, m, p and A are as defined in claim 1; and compounds that are pharmaceutically acceptable salts thereof.

3. A compound of the Formula Ic:

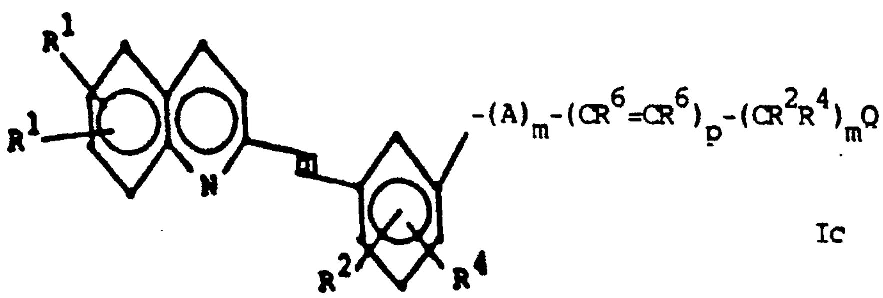

Ic

in which the variables are as defined in claim 2, and compounds that are pharmaceutically acceptable salts thereof.

4. The following compounds as claimed in claim 1:
4-(2-(quinolin-2-yl)ethenyl)benzoic acid methyl ester;
4-(2-(quinolin-2-yl)ethenyl)benzoic acid;
3-(2-(quinolin-2-yl)ethenyl)benzenepropanoic acid ethyl ester;
3-(2-(Quinolin-2-yl)ethenyl)benzenepropanoic acid;
3-(2-(quinolin-2-yl)ethenyl)-γ-oxo-β-methylbenzene butanoic acid methyl ester;
3-(2-(quinolin-2-yl)ethenyl)-γ-hydroxy-β-methylbenzenebutanoic acid methyl ester;
[βR,γS and βS,γR]-3-(2-quinolin-2-yl)-ethenyl)-γ-hydroxy-β-methylbenzenebutanoic acid lactone;

[$\beta$S,$\gamma$S and $\beta$R,$\gamma$R]-3-(2-quinolin-2-yl)-ethenyl)-$\gamma$-hydroxy-$\beta$-methylbenzenebutanoic acid lactone;
[$\beta$R,$\gamma$S and $\beta$S,$\gamma$R]-3-(2-(Quinolin-2-yl)ethenyl)-$\gamma$-hydroxy-$\beta$-methylbenzenebutanoic acid sodium salt;
[$\beta$S,$\gamma$S and $\beta$R,$\gamma$R]-3-(2-quinolin-2-yl)ethenyl)-$\gamma$-hydroxy-$\beta$-methylbenzenebutanoic acid sodium salt;
3-(2-(7-Chloroquinolin-2-yl)ethenyl)-$\beta$-methylbenzenepropanoic acid;

**5.** A pharmaceutical composition useful in antagonizing leukotriene action in mammals comprising a compound as claimed in any one of claims 1 to 4 in an amount effective as a leukotriene antagonist and a pharmaceutically acceptable carrier.

**6.** A composition as claimed in claim 5 additionally comprising an effective amount of a second active ingredient that is a non-steroidal anti-inflammatory drug; a peripheral analgesic agent; cyclooxygenase inhibitor; a leukotriene antagonist; a leukotriene inhibitor; an $H_2$-receptor antagonist; an antihistaminic agent; a prostaglandin antagonist or a thromboxane antagonist.

**7.** A compound as claimed in any one of claims 1 to 4 for use in preventing the synthesis, the action, or the release of SRS-A or leukotriene $D_4$ in mammals.

**8.** A compound as claimed in any one of claims 1 to 4 for use in treating inflammatory diseases of the eye in mammals.

**Claims for the following Contracting State : AT**

**1.** A method for preparing a novel compound of the formula Ia:

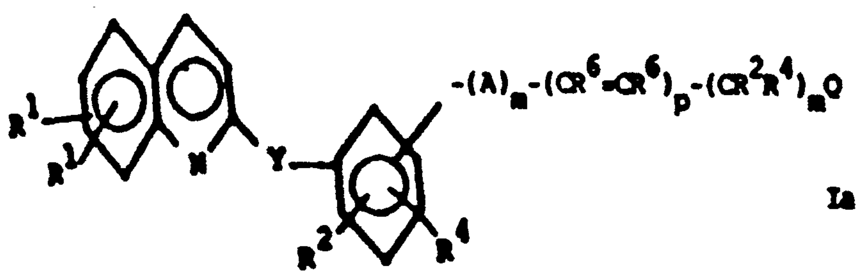

Ia ,

in which

| | |
|---|---|
| Y is | $-(CR^2=CR^2)_n-$ or $-(C\equiv C)_n-$; |
| $R^1$ is | H, halogen $C_{1-8}$ alkyl, $C_{2-8}$ alkenyl, $C_{2-8}$ alkynyl, $-CF_3$, $-OR^2$, $-SR^2$, $SOR^2$, $-SO_2R^2$, $-NR^2R^2$, -CHO, $-COOR^2$, $-(C=O)R^2$, $-C(OH)R^2R^2$, -CN, $-NO_2$, substituted or $-(C=O)R^2$, $-C(OH)R^2R^2$, -CN, $NO_2$ substituted or unsubstituted phenyl, substituted or unsubstituted benzyl, or substituted or unsubstituted phenethyl; |
| $R^2$ is | H, $C_{1-8}$ alkyl, $C_{2-8}$ alkenyl, $C_{2-8}$ alkynyl, $-CF_3$, substituted or unsubstituted phenyl, substituted or unsubstited benzyl, or substituted or unsubstituted phenethyl: |
| $R^4$ is | H, halogen, $-NO_2$, -CN, $-OR^2$, $-SR^2$, $-NR^2R^2$ or $C_{1-8}$ alkyl; |
| m is | 0 or an integer not exceeding 8; |
| n is | 1 or 2; |
| p is | 0 or 1; |
| A is | $-CR^4R^4-$ or $=C=O$; |
| Q is | $-COOR^2$, tetrazole, |

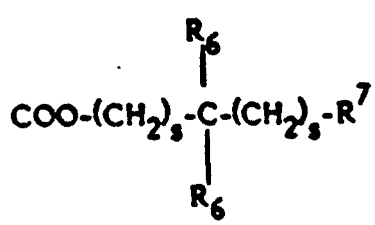

(where s is 0 or an integer not exceeding 3, $R^6$ is hydrogen or $C_{1-4}$ alkyl, and $R^7$ is $OR^8$, $SR^8$ or $\overline{N}HR^8$, where $R^8$ is a $C_{1-21}$ hydrocarbon radical or a $C_{1-21}$ acyl radical of an organic acyclic or monocyclic carboxylic acid containing not more than one hetero atom in the ring, or $R^7$ is a monocyclic or bicyclic heterocyclic radical containing 3 to 12 nuclear carbons and 1 or 2 N, S and/or O hetero atoms, each ring having 5 or 6 atoms), $-CONHSO_2R^{11}$, $-CONR^{10}R^{10}$, or (but only when the sum of m, n and p is greater than 0) $-NHSO_2R^{11}$; or if Q is COOH and $R^3$ contains an $R^4$ that is -OH, then Q and $R^4$ and the carbons through which they are attached may form a lactone ring with loss of water;

$R^{10}$ is H, $C_{1-6}$ alkyl, $-(C=O)R^{11}$, unsubstituted phenyl or unsubstituted benzyl; and

$R^{11}$ is H, $C_{1-8}$ alkyl, $C_{2-8}$ alkenyl, $C_{2-8}$ alkynyl, $-CF_3$, or unsubstituted phenyl, benzyl, or phenethyl;

substituted phenyl, benzyl or phenethyl means phenyl, benzyl or phenethyl having one or two substituents on the benzene ring selected from $C_{1-6}$ alkyl, $NO_2$, $SCF_3$, halogen, $COR^9$, CN, and $CF_3$, where $R^9$ is $-OR^{10}$, $-SR^{10}$, or $-NR^{10}R^{10}$;

provided that where two or more of any variable are present, each is the same as or different from the other(s);

or the pharmaceutically acceptable salts thereof,

comprising:

heating an aniline derivative of formula II:

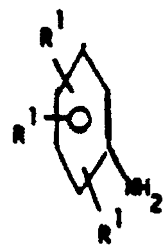

II

with a crotonaldehyde in presence of a strong mineral acid to provide the substituted quinaldine derivative of formula III:

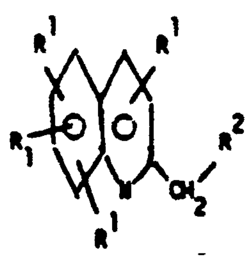 ;

III

separating the possibly obtained regioisomers of the quinaldine derivative of formula III;

treating the derivative of formula III with a halogen source in the presence of light to provide the quinaldine of formula IV:

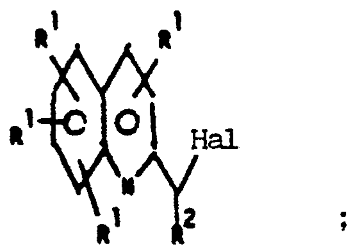

IV

treating the quinaldine of formula IV with triphenylphosphine in an inert solvent to provide the phosphonium salt of formula V:

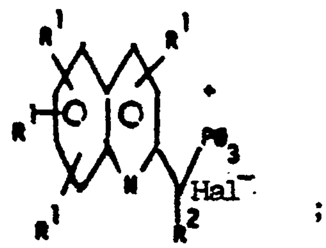

V

reacting the quinaldine derivative of formula V with a strong base in an inert solvent at low temperature;

reacting the resulting ylid with the dialdehyde of formula VI:

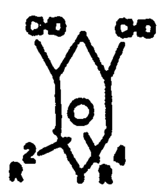

VI

to provide the vinyl quinoline of formula VII:

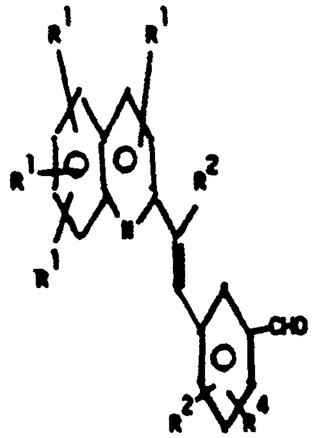

;

VII

treating the quinoline of formula VII with a ylid of formula VIII:

$\phi_3P=CH\text{-}(CH_2)_{(m-2)}\text{-}Q$ VIII

to provide the adduct of formula IX:

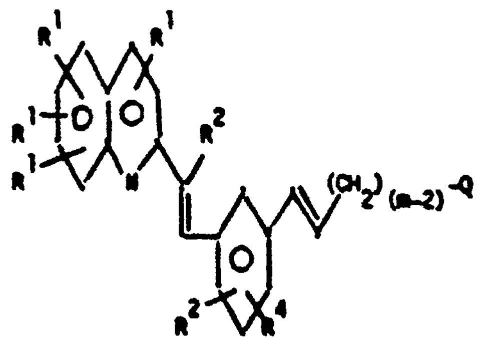

IX

and optionally treating the adduct of formula IX with hydrogen in presence of a suitable catalyst to provide a 2-styryl quinoline derivative of formula X:

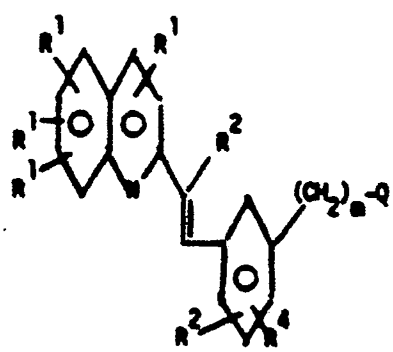

,

X

wherein $R^1$, $R^2$, $R^4$, Q and m are as defined in claim 1.

2. A method according to claim 1, wherein the dialdehyde of formula VI is reacted with the ylid of formula VIII and after reducing of the double bond the thus obtained compound is reacted with the quinaldine derivative of formula V to provide the 2-styryl quinoline derivative of formula X.

3. A method for preparing a compound as defined in claim 1, comprising:

reacting the aldehyde of formula VII:

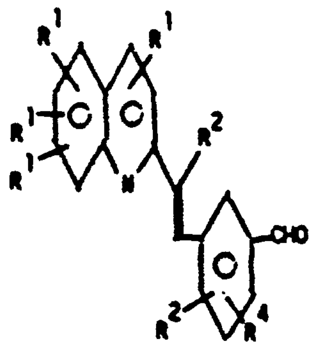

VII

with a Grignard reagent of formula XI:

$R^2CH_2MgHal$ XI

or an equivalent organolithium reagent to provide the alcohol of formula XII:

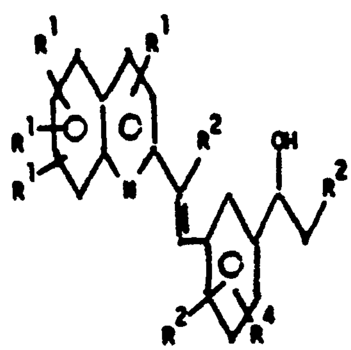

;

XII

oxidising the alcohol of formula XII with a suitable oxidizing agent to provide the ketone of formula XIII:

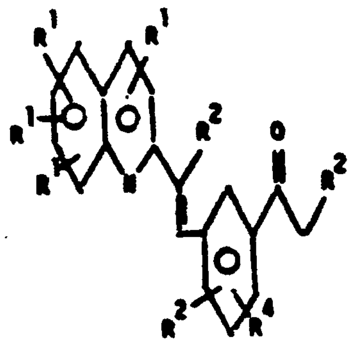

;

XIII

deprotonating the ketone of formula XIII with a strong base in an inert solvent at low temperature;

reacting the thus obtained enolate with an alkanoic acid, ester, nitrile or tetrazole, terminally substituted with a leaving group, to provide the adduct of formula XIV:

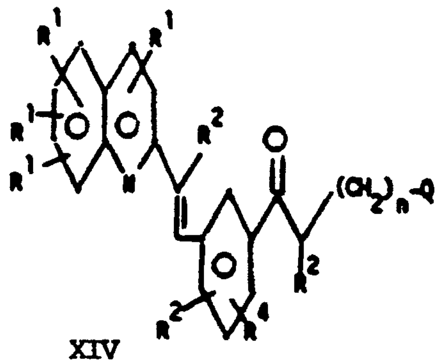

XIV

and optionally reducing the adduct of formula XIV with a suitable reducing agent to provide the quinoline alcohol of formula XV:

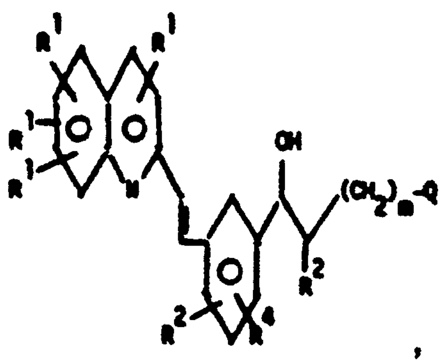

,

XV

wherein $R^1$, $R^2$, $R^4$, Q and m are as defined in claim 1.

**4.** A method for preparing a compound as defined in claim 1, comprising:

heating a quinoline derivative of formula III:

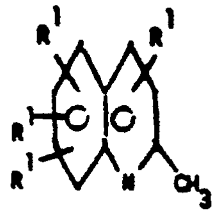

III

with a dialdehyde of formula VI:

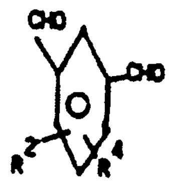

VI

in presence of a dehydrating agent to provide a 2-styryl quinoline aldehyde of formula VII:

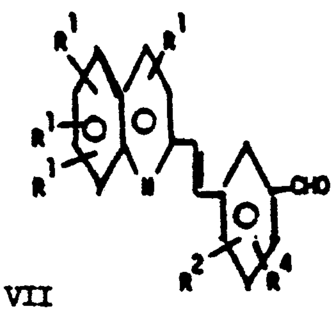

VII

and treating the 2-styryl quinoline aldehyde of formula VII according to any one of Claims 1 to 3 to provide the corresponding compounds, wherein $R^1$, $R^2$, $R^4$, Q and m are as defined in claim 1.

**5.** A method for preparing a compound as defined in claim 1, comprising:

condensing a dialdehyde of formula VI:

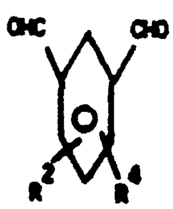

VI

with an ylid of formula VIII:

$\phi_3P=CH\text{-}(CH2)_{m\text{-}2}\text{-}Q$ VIII

to provide an olefinic aldehyde of formula XVI:

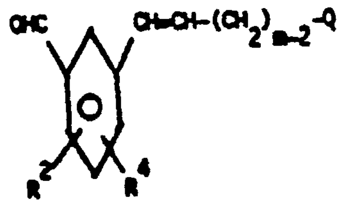

;

XVI

reducing the olefinic aldehyde of formula XVI with hydrogen in presence of a suitable catalyst to provide the aldehyde of formula XVII:

XVII

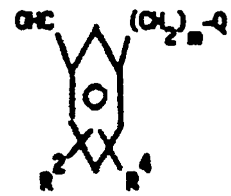

and condensing the aldehyde of formula XVII with a quinaldine of formula III:

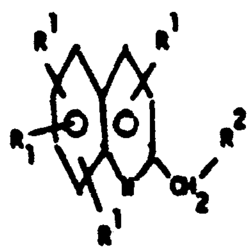

III

in presence of a dehydrating agent to provide a compound of formula X:

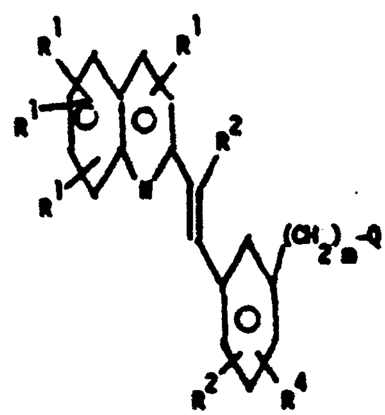

,

X

wherein $R^1$, $R^2$, $R^4$, Q and m are as defined in claim 1.

**6.** A method for preparing a compound as defind in claim 1, comprising

half-reducing and protecting the thus formed alcohol group of a dialdehyde of formula VI:

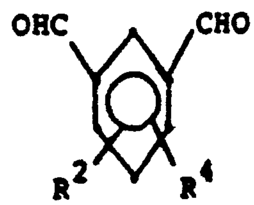

VI

to provide a compound of formula XVIII

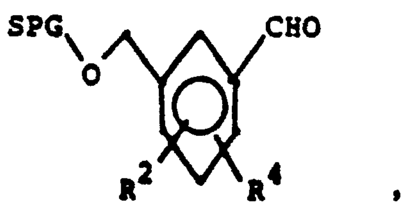

XVIII,

wherein SPG is a silyl protecting group;

reacting the compound of formula XVIII with a compound of formula XIXa:

$R^2$-Li XIXa,

wherein $R^2$ is other than hydrogen, to provide a compound of formula XX:

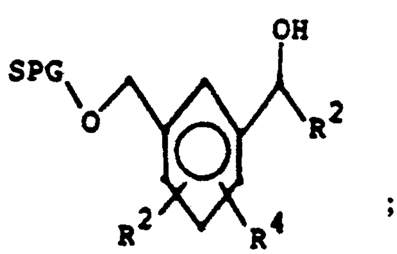

;

XX

oxidising the compound of formula XX with a suitable oxidizing agent to provide a compound of formula XXI:

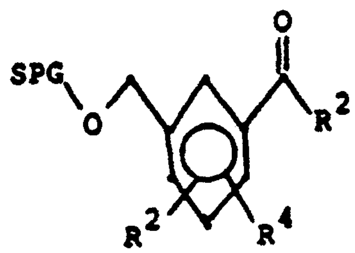

;

XXI

condensing the compound of formula XXI with the silyl ester of formula XXII:

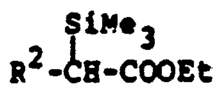

;

XXII

reducing the thus obtained α,β-unsaturated ester to the compound of formula XXIII:

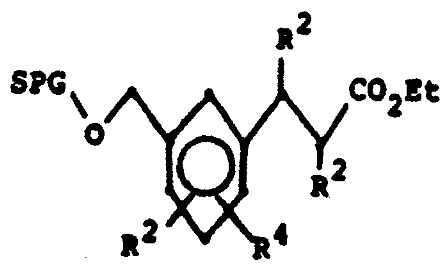

;

XXIII

converting the protected alcohol group of the compound of formula XXIII into an aldehyde group by known methodology to provide the aldehyde of formula XXIV:

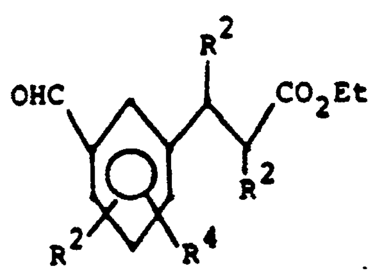

;

XXIV

condensing the aldehyde of formula XXIV with a compound of formula III:

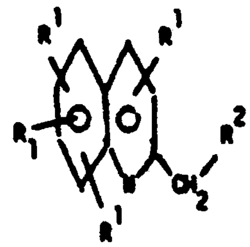

III

in presence of a dehydrating agent to provide the compound of formula XXV:

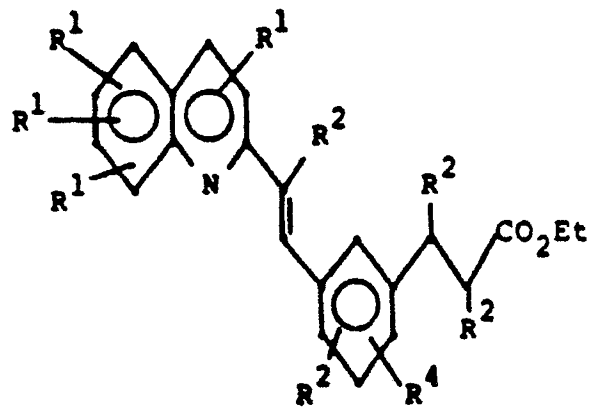

XXV

and optionally hydrolysing the compound of formula XXV to provide the compound of formula XXVI:

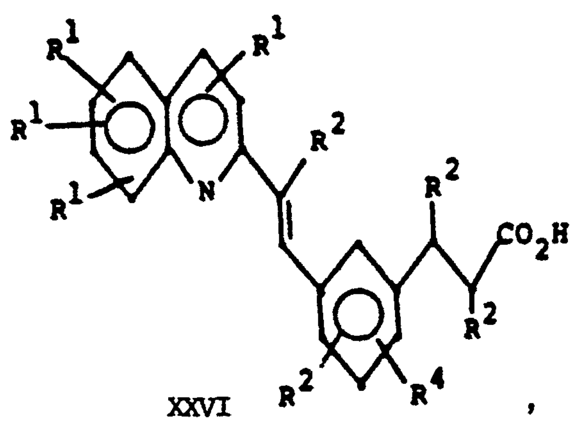

XXVI ,

wherein $R^1$, $R^2$ and $R^4$ are as defined in claim 1.

**7.** A method according to any one of claims 1 to 6 as applied to the preparation of a compound of the formula Ib:

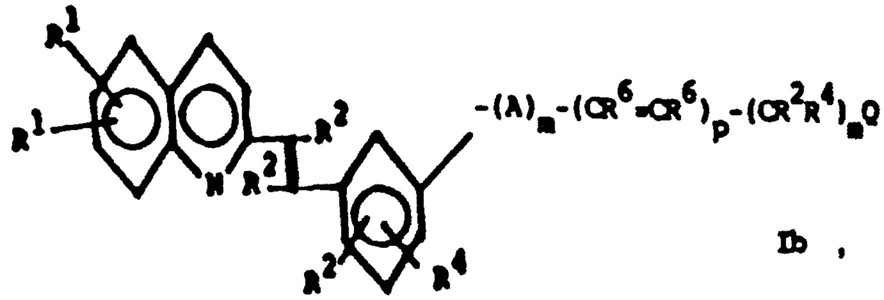

Ib ,

in which $R^1$ is H, halogen, $CH_3$, $-CF_3$ or $SCF_3$; $R^2$ is H, $C_{1-3}$ alky], $C_{2-3}$ alkenyl, or $-CF_3$; and $R^4$, $R^6$, m, p and A are as defined in claim 1; and of compounds that are pharmaceutically acceptable salts thereof.

**8.** A method according to any one of claims 1 to 6 as applied to the preparation of a compound of the formula Ic:

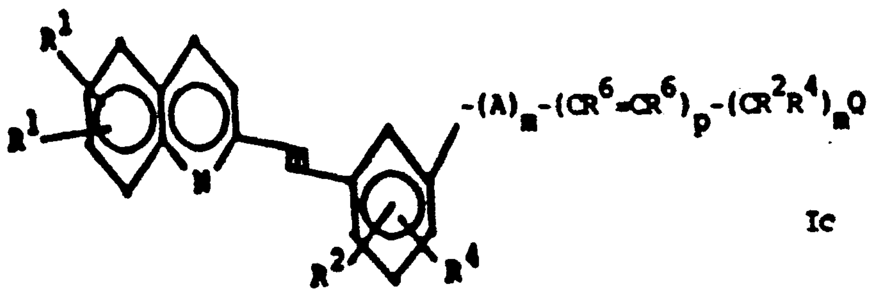

Ic ,

in which the variables are as defined in claim 7, and of compounds that are pharmaceutically acceptable salts thereof.

**9.** A method according to any one of claims 1 to 6 as applied to the preparation of the following compounds as defined in claim 1:
4-(2-(quinolin-2-yl)ethenyl)benzoic acid methyl ester;
4-(2-(quinolin-2-yl)ethenyl)benzoic acid;
3-(2-(quinolin-2-yl)ethenyl)benzenepropanoic acid ethyl ester;
3-(2-(Quinolin-2-yl)ethenyl)benzenepropanoic acid;
3-(2-(quinolin-2-yl)ethenyl)-γ-oxo-β-methylbenzene butanoic acid methyl ester;
3-(2-(quinolin-2-yl)ethenyl)-γ-hydroxy-β-methyl-benzenebutanoic acid methyl ester;
[βR,γS and βS,γR]-3-(2-quinolin-2-yl)-ethenyl)-γ-hydroxy-β-methylbenzenebutanoic acid lactone;
[βS,γS and βR,γR]-3-(2-quinolin-2-yl)-ethenyl)-γ-hydroxy-β-methylbenzenebutanoic acid lactone;
[βR,γS and βS,γR]-3-(2-(Quinolin-2-yl)ethenyl)-γ-hydroxy-β-methylbenzenebutanoic acid sodium salt;
[βS,γS and βR,γR]-3-(2-quinolin-2-yl)ethenyl)-γ-hydroxy-β-methylbenzenebutanoic acid sodium salt;
3-(2-(7-Chloroquinolin-2-yl)ethenyl)-β-methylbenzene-propanoic acid;

**10.** A process for preparing a pharmaceutical composition useful in antagonizing leukotriene action in mammals, comprising admixing of a compound as prepared in any one of the claims 1 to 9 in an amount effective as a leukotriene antagonist with a pharmaceutically acceptable carrier.

**11.** A process for preparing a pharmaceutical composition according to claim 10, which additionally comprises admixing with a effective amount of a second active ingredient that is a non-steroidal anti-inflammatory drug; a peripheral analgesic agent; a cyclooxygenase inhibitor; a leukotriene antagonist; a leukotriene inhibitor; an $H_2$-receptor antagonist; an antihistaminic agent; a prostaglandin antagonist or a thromboxane antagonist.

**12.** The use of a compound as prepared in any one of claims 1 to 9 for preparing a composition for use in

preventing the synthesis, the action or the release of SRS-A or leukotriene $D_4$ in mammals.

13. The use of a compound as prepared in any one of claims 1 to 9 for preparing a composition for use in treating inflammatory diseases of the eye in mammals.

**Revendications**

**Revendications pour les Etats contractants suivants : BE, CH, DE, FR, GB, IT, LI, LU, NL, SE**

1. Composé de formule Ia :

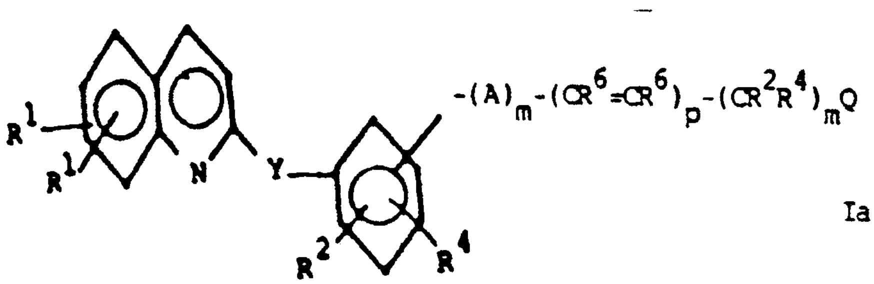

Ia

dans laquelle

Y est $-(CR^2=CR^2)_n-$ ou $-(C\equiv C)_n-$;

$R^1$ est H, un halogène, un groupe alkyle en $C_1$-$C_8$, alcényle en $C_2$-$C_8$, alcynyle en $C_2$-$C_8$, $-CF_3$, $-OR^2$, $-SR^2$, $-SOR^2$, $-SO_2R^2$, $-NR^2R^2$, -CHO, $-COOR^2$, $-(C=O)R^2$, $-C(OH)R^2R^2$, -CN, $-NO_2$, un groupe phényle substitué ou non substitué, un groupe benzyle substitué ou non substitué, ou un groupe phénéthyle substitué ou non substitué;

$R^2$ est H, un groupe alkyle en $C_1$-$C_8$, alcényle en $C_2$-$C_8$, alcynyle en $C_2$-$C_8$, $-CF_3$, un groupe phényle substitué ou non substitué, un groupe benzyle substitué ou non substitué ou un groupe phénéthyle substitué ou non substitué;

$R^4$ est H, un halogène, $-NO_2$, -CN, $-OR^2$, $-SR^2$, $-NR^2R^2$ ou un groupe alkyle en $C_1$-$C_8$;

m est 0 ou un nombre entier ne dépassant pas 8;

n est 1 ou 2;

p est 0 ou 1;

A est $-CR^4R^4-$ ou $=C=O$;

Q est $-COOR^2$, un tétrazole,

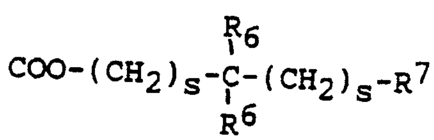

(où s est 0 ou un nombre entier ne dépassant pas 3, $R^6$ est un hydrogène ou un groupe alkyle en $C_1$-$C_4$, et $R^7$ est $OR^8$, $SR^8$ ou $NHR^8$, où $R^8$ est un radical hydrocarboné en $C_1$-$C_{21}$ ou un radical acyle en $C_1$-$C_{21}$ d'un acide carboxylique organique acyclique ou monocyclique ne contenant pas plus d'un hétéroatome dans le noyau, ou $R^7$ est un radical hétérocyclique monocyclique ou bicyclique contenant 3 à 12 atomes de carbone dans le noyau et 1 ou 2 hétéroatomes N, S et/ou O, chaque noyau comportant 5 ou 6 atomes), $-CONHSO_2R^{11}$, $-CONR^{10}R^{10}$, ou (mais seulement lorsque la somme des m, m et p est supérieure à 0) $-NHSO_2R^{11}$; ou bien, si Q est COOH et $R^3$ contient un $R^4$ qui est -OH, Q et $R^4$ et les atomes de carbone par l'intermédiaire desquels ils sont fixés peuvent former un noyau lactone avec perte d'eau;

$R^{10}$ est H, un groupe alkyle en $C_1$-$C_6$, $-(C=O)R^{11}$, ou un groupe phényle non substitué ou benzyle non substitué; et

$R^{11}$ est H, un groupe alkyle en $C_1$-$C_8$, alcényle en $C_2$-$C_8$, alcynyle en $C_2$-$C_8$, $-CF_3$ ou un groupe phényle, benzyle ou phénéthyle non substitué;

les termes groupes phényle, benzyle ou phénéthyle substitués signifient des groupes phényle, benzyle

ou phénéthyle comportant un ou deux substituants sur le noyau benzène choisis parmi un groupe alkyle en $C_1$-$C_6$, $NO_2$, $SCF_3$, un halogène, $COR^9$, CN et $CF_3$, où $R^9$ est $-OR^{10}$, $-SR^{10}$ ou $-NR^{10}R^{10}$;
à condition que, lorsque deux ou plus de deux variables sont présentes, chacune soit identique ou différente de la ou des autres;
ou sont leurs sels pharmaceutiquement acceptables.

**2.** Composé de formule Ib :

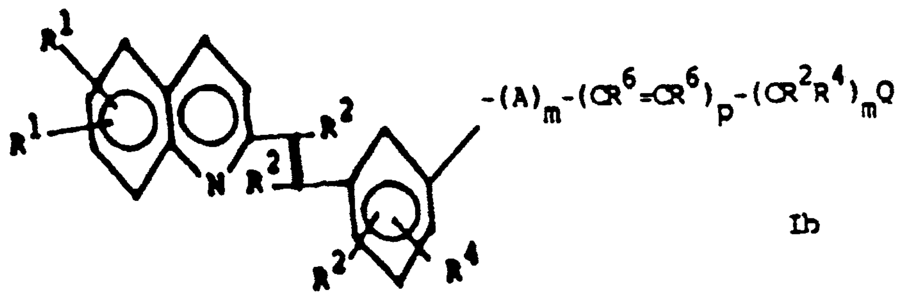

Ib

dans laquelle $R^1$ est H, un halogène, $CH_3$, $-CF_3$ ou $SCF_3$; $R^2$ est H, un groupe alkyle en $C_1$-$C_3$, alcényle en $C_2$-$C_3$ ou $-CF_3$; et $R^4$, $R^6$, m, p et A sont tels que définis dans la revendication 1; et composés qui sont leurs sels pharmaceutiquement acceptables.

**3.** Composé de formule Ic :

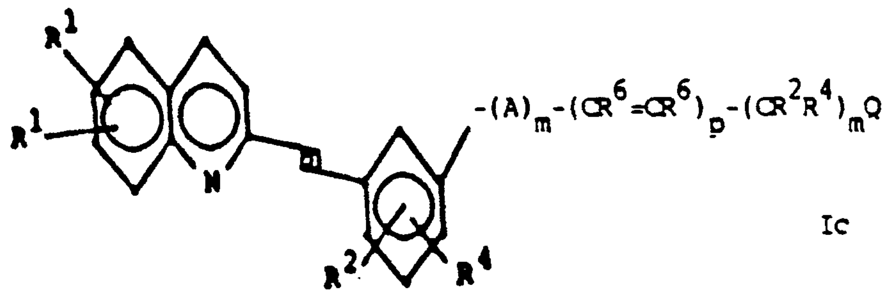

Ic

dans laquelle les variables sont telles que définies dans la revendication 2, et composés qui sont leurs sels pharmaceutiquement acceptables.

**4.** Composés suivants selon la revendication 1 :
ester méthylique de l'acide 4-(2-(quinoléine-2-yl)éthényl)benzoïque;
acide 4-(2-(quinoléine-2-yl)éthényl)benzoïque;
ester éthylique de l'acide 3-(2-(quinoléine-2-yl)éthényl)benzènepropanoïque;
acide 3-(2-(quinoléine-2-yl)éthényl)benzènepropanoïque;
ester méthylique de l'acide 3-(2-(quinoléine-2-yl)éthényl)-γ-oxo-β-méthylbenzènebutanoïque;
ester méthylique de l'acide 3-(2-(quinoléine-2-yl)éthényl)-γ-hydroxy-β-méthylbenzènebutanoïque;
lactone de l'acide [βR,γS et βS,γR]-3-(2-quinoléine-2-yl)éthényl)-γ-hydroxy-β-méthylbenzènebutanoïque;
lactone de l'acide [βS,γS et βR,γR]-3-(2-quinoléine-2-yl)éthényl)-γ-hydroxy-β-méthylbenzènebutanoïque;
sel de sodium de l'acide [βR,γS et βS,γR]-3-(2-(quinoléine-2-yl)éthényl)-γ-hydroxy-β-méthylbenzènebutanoïque;
sel de sodium de l'acide [βS,γS et βR,γR]-3-(2-(quinoléine-2-yl)éthényl)-γ-hydroxy-β-méthylbenzènebutanoïque;
acide 3-(2-(7-chloroquinoléine-2-yl)éthényl)-β-méthylbenzènepropanoïque;

**5.** Composition pharmaceutique utile pour antagoniser l'action des leucotriènes chez les mammifères,

comprenant un composé selon l'une quelconque des revendications 1 à 4, en une quantité efficace comme antagoniste des leucotriènes et un véhicule pharmaceutiquement acceptable.

6. Composition selon la revendication 5, comprenant en outre une quantité efficace d'un second ingrédient actif qui est un anti-inflammatoire non stéroïdien, un agent analgésique périphérique, un inhibiteur de cyclooxygénase, un antagoniste des leucotriènes, un inhibiteur des leucotriènes, un antagoniste des récepteurs $H_2$, un agent antihistaminique, un antagoniste de la prostaglandine ou un antagoniste du thromboxane.

7. Composé selon l'une quelconque des revendications 1 à 4, utilisable pour empêcher la synthèse, l'action ou la libération de la SRS-A ou du leucotriène $D_4$ chez les mammifères.

8. Composé selon l'une quelconque des revendications 1 à 4, utilisable pour traiter les maladies inflammatoires de l'oeil des mammifères.

**Revendications pour l'Etat contractant suivant : AT**

1. Procédé de préparation d'un nouveau composé de formule Ia :

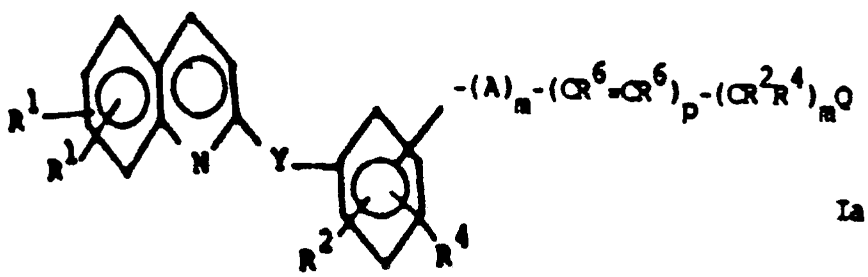

Ia ,

dans laquelle

| | |
|---|---|
| Y est | $(CR^2=CR^2)_n$- ou -$(C\equiv C)_n$-; |
| $R^1$ est | H, un halogène, un groupe alkyle en $C_1$-$C_8$, alcényle en $C_2$-$C_8$, alcynyle en $C_2$-$C_8$, -$CF_3$, -$OR^2$, -$SR^2$ -$SOR^2$, -$SO_2R^2$, -$NR^2R^2$, -CHO, -$COOR^2$, -$(C=O)R^2$, -$C(OH)R^2R^2$, -CN, -$NO_2$, un groupe phényle substitué ou non substitué, un groupe benzyle substitué ou non substitué, ou un groupe phénéthyle substitué ou non substitué; |
| $R^2$ est | H, un groupe alkyle en $C_1$-$C_8$, alcényle en $C_2$-$C_8$, alcynyle en $C_2$-$C_8$, -$CF_3$, un groupe phényle substitué ou non substitué, un groupe benzyle substitué ou non substitué ou un groupe phénéthyle substitué ou non substitué; |
| $R^4$ est | H un halogène, -$NO_2$, -CN, -$OR^2$, -$SR^2$, -$NR^2R^2$ ou un groupe alkyle en $C_1$-$C_8$; |
| m est | 0 ou un nombre entier ne dépassant pas 8; |
| n est | 1 ou 2; |
| p est | 0 ou 1; |
| A est | -$CR^4R^4$- ou =C=O; |
| Q est | -$COOR^2$, un tétrazole, |

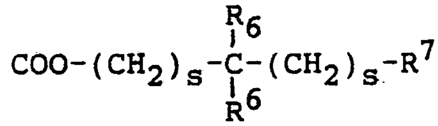

(où s est 0 ou un nombre entier ne dépassant pas 3, $R^6$ est un hydrogène ou un groupe alkyle en $C_1$-$C_4$, et $R^7$ est $OR^8$, $SR^8$ ou $NHR^8$, où $R^8$ est un radical hydrocarboné en $C_1$-$C_{21}$ ou un radical acyle en $C_1$-$C_{21}$ d'un acide carboxylique organique acyclique ou monocyclique ne contenant pas plus d'un hétéroatome dans le noyau, ou $R^7$ est un radical hétérocyclique monocyclique ou bicyclique contenant 3 à 12 atomes de carbone dans le noyau et 1 ou 2 hétéroatomes N, S et/ou O, chaque noyau comportant 5 ou 6 atomes), -$CONHSO_2R^{11}$, -$CONR^{10}R^{10}$, ou (mais seulement lorsque la somme des m, m

et p est supérieure à 0) $-NHSO_2R^{11}$; ou bien, si Q est COOH et $R^3$ contient un $R^4$ qui est -OH, Q et $R^4$ et les atomes de carbone par l'intermédiaire desquels ils sont fixés peuvent former un noyau lactone avec perte d'eau;

$R^{10}$ est H, un groupe alkyle en $C_1$-$C_6$, $-(C=O)R^{11}$, ou un groupe phényle non substitué ou benzyle non substitué; et

$R^{11}$ est H, un groupe alkyle en $C_1$-$C_8$, alcényle en $C_2$-$C_8$, alcynyle en $C_2$-$C_8$, $-CF_3$ ou un groupe phényle, benzyle ou phénéthyle non substitué;

les termes groupes phényle, benzyle ou phénéthyle substitués signifient des groupes phényle, benzyle ou phénéthyle comportant un ou deux substituants sur le noyau benzène choisis parmi un groupe alkyle en $C_1$-$C_6$, $NO_2$, $SCF_3$, un halogène, $COR^9$, CN et $CF_3$, où $R^9$ est $-OR^{10}$, $-SR^{10}$ ou $-NR^{10}R^{10}$;

à condition que, lorsque deux ou plus de deux variables sont présentes, chacune soit identique ou différente de la ou des autres;

ou de ses sels pharmaceutiquement acceptables,

qui comprend les étapes qui consistent à :

chauffer un dérivé aniline de formule II :

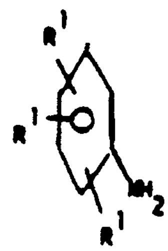

II

avec un crotonaldéhyde, en présence d'un acide minéral fort, pour obtenir le dérivé quinaldine substitué de formule III :

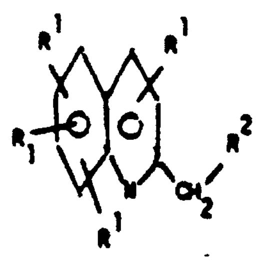

;

III

séparer les régioisomères éventuellement obtenus du dérivé quinaldine de formule III;

traiter le dérivé de formule III avec une source d'halogène, en présence de lumière, pour obtenir la quinaldine de formule IV :

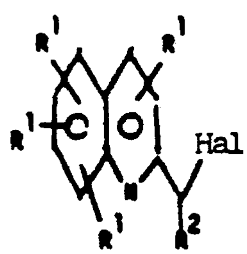

;

IV

traiter la quinaldine de formule IV avec la triphénylphosphine, dans un solvant inerte, pour obtenir le sel de phosphonium de formule V :

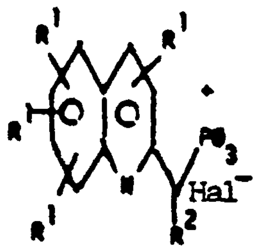

;

V

faire réagir le dérivé quinaldine de formule V avec une base forte, dans un solvant inerte, à basse température;

faire réagir l'ylide résultant avec le dialdéhyde de formule VI :

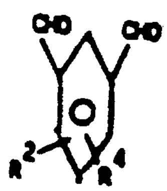

VI

pour obtenir la vinylquinoléine de fomule VII :

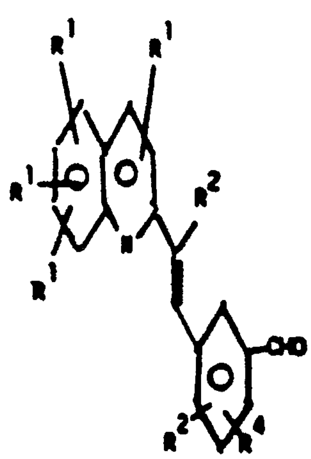

;

VII

traiter la quinoléine de formule VII avec un ylide de formule VIII :

$\phi_3P=CH-(CH_2)_{(m-2)}-Q$ VIII

pour obtenir le composé d'addition de formule IX :

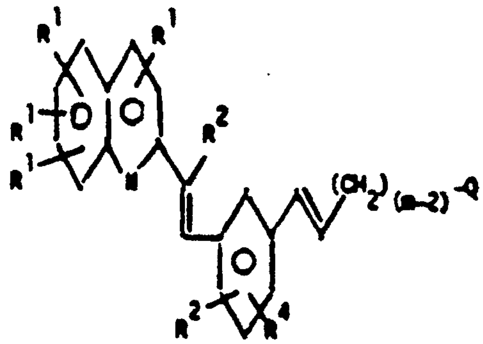

IX

et, éventuellement, traiter le composé d'addition de formule IX avec de l'hydrogène, en présence d'un catalyseur convenable, pour obtenir le dérivé 2-styrylquinoléine de formule X :

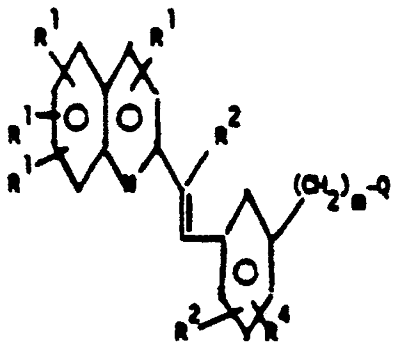

,

X

dans laquelle $R^1$, $R^2$, $R^4$, Q et m sont tels que définis dans la revendication 1.

2. Procédé selon la revendication 1, dans lequel on fait réagir le dialdéhyde de formule VI avec l'ylide de formule VIII et, après avoir réduit la double liaison, on fait réagir le composé ainsi obtenu avec le dérivé quinaldine de formule V pour obtenir le dérivé 2-styrylquinoléine de formule X.

3. Procédé de préparation d'un composé tel que défini dans la revendication 1, comprenant les étapes qui consistent à :

faire réagir l'aldéhyde de formule VII :

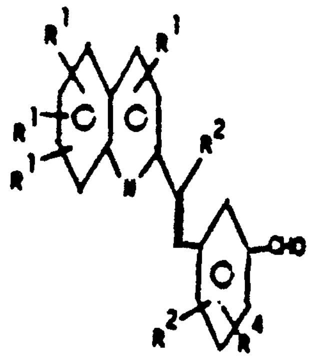

VII

avec un réactif de Grignard de formule XI :

$R^2CH_2MgHal$ XI

ou un réactif organolithié équivalent, pour obtenir l'alcool de formule XII :

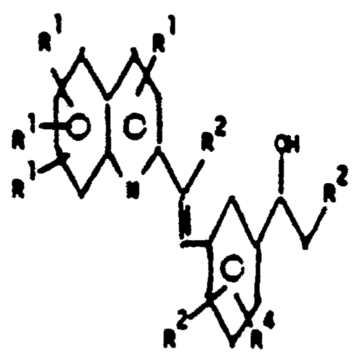

;

XII

oxyder l'alcool de formule XII avec un agent oxydant convenable, pour obtenir la cétone de formule XIII :

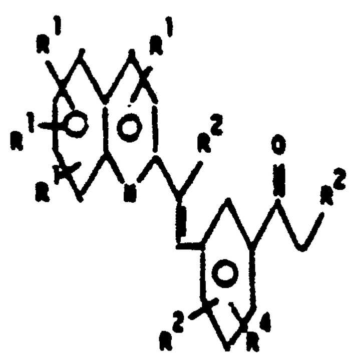

;

XIII

déprotoner la cétone de formule XIII avec une base forte, dans un solvant inerte, à basse température;

faire réagir l'énolate ainsi obtenu avec un acide alcanoïque, un ester, un nitrile ou un tétrazole, substitué à son extrémité par un groupe partant, pour obtenir le composé d'addition de formule XIV :

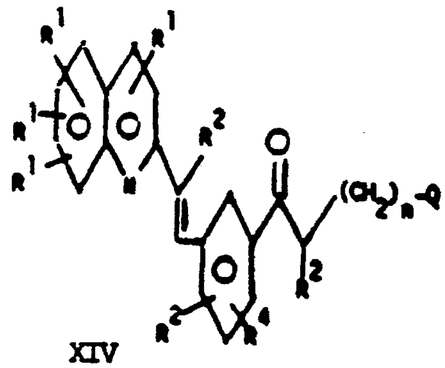

XIV

et, éventuellement, réduire le composé d'addition de formule XIV avec un agent réducteur convenable, pour obtenir le quinoléine-alcool de formule XV :

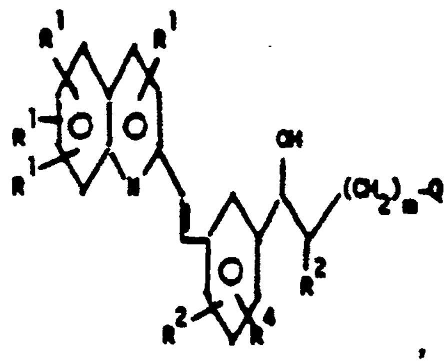

XV

dans laquelle $R^1$, $R^2$, $R^4$, Q et m sont tels que définis dans la revendication 1.

**4.** Procédé de préparation d'un composé tel que défini dans la revendication 1, comprenant les étapes qui consistent à :

chauffer un dérivé quinoléine de formule III :

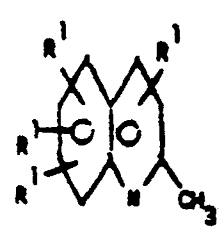

III

avec un dialdéhyde de formule VI :

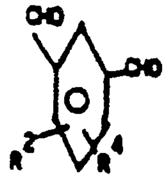

VI

en présence d'un agent déshydratant, pour obtenir le 2-styrylquinoléine-aldéhyde de formule VII :

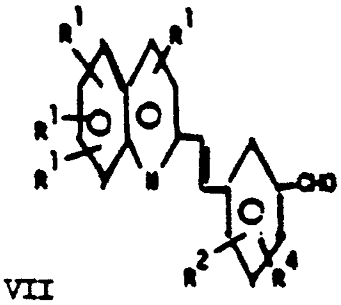

VII

et traiter le 2-styrylquinoléine-aldéhyde de formule VII selon l'une quelconque des revendications 1 à 3, pour obtenir les composés correspondants, dans lesquels $R^1$, $R^2$, $R^4$, Q et m sont tels que définis dans la revendication 1.

5. Procédé de préparation d'un composé tel que défini dans la revendication 1, comprenant les étapes qui consistent à :
condenser un dialdéhyde de formule VI :

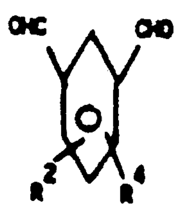

VI

avec un ylide de formule VIII :

$\phi_3P=CH-(CH2)_{m-2}-Q$ VIII

pour obtenir un aldéhyde oléfinique de formule XVI :

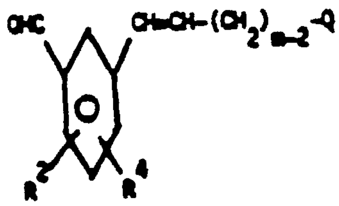

;

XVI

réduire l'aldéhyde oléfinique de formule XVI avec de l'hydrogène, en présence d'un catalyseur convenable, pour obtenir l'aldéhyde de formule XVII :

XVII 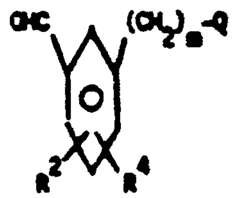

et condenser l'aldéhyde de formule XVII avec une quinaldine de formule III :

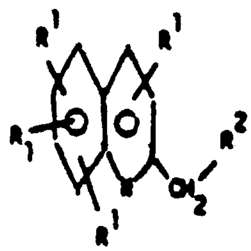

III

en présence d'un agent déshydratant, pour obtenir un composé de formule X :

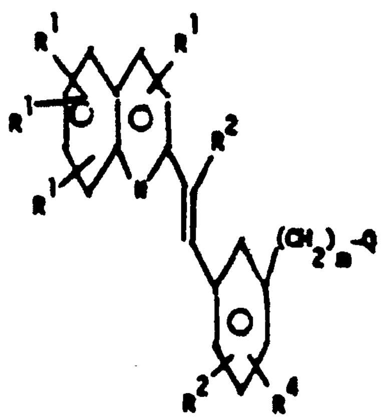

,

X

dans laquelle $R^1$, $R^2$, $R^4$, Q et m sont tels que définis dans la revendication 1.

6. Procédé de préparation d'un composé tel que défini dans la revendication 1, comprenant les étapes qui

consistent à :

effectuer la semi-réduction, et la protection du groupe alcool ainsi formé, d'un dialdéhyde de formule VI :

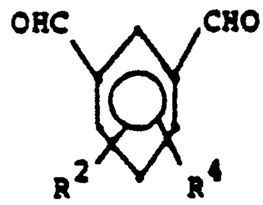

VI

pour obtenir un composé de formule XVIII :

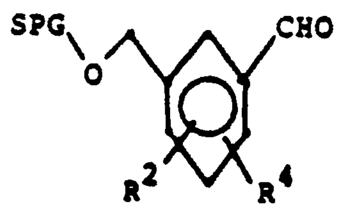

,

XVIII,

dans laquelle SPG est un groupe protecteur de silyle;

faire réagir le composé de formule XVIII avec un composé de formule XIXa :

$R^2$-Li XIXa

dans laquelle $R^2$ est autre qu'un atome d'hydrogène, pour obtenir un composé de formule XX :

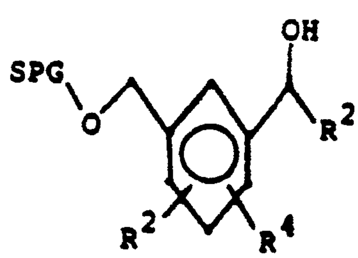

;

XX

oxyder le composé de formule XX avec un agent oxydant convenable, pour obtenir un composé de formule XXI :

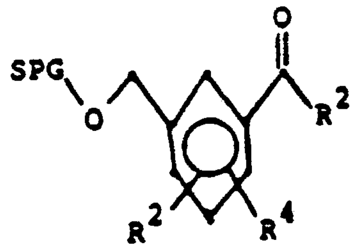

;

XXI

condenser le composé de formule XXI avec l'ester silylique de formule XXII :

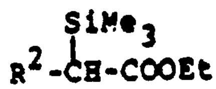

;

XXII

réduire l'ester $\alpha,\beta$-insaturé ainsi obtenu en composé de formule XXIII :

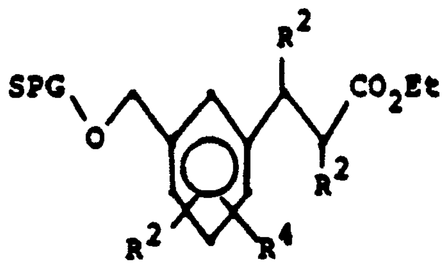

;

XXIII

transformer le groupe alcool protégé du composé de formule XXIII en groupe aldéhyde par une méthodologie connue, pour obtenir l'aldéhyde de formule XXIV :

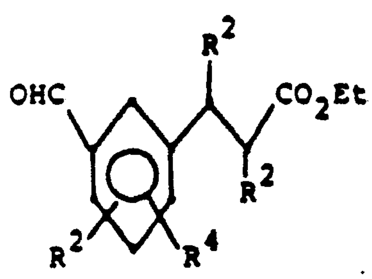

;

XXIV

condenser l'aldéhyde de formule XXIV avec un composé de formule III :

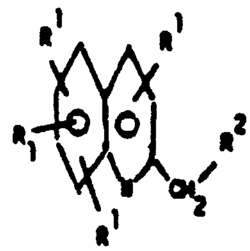

III

en présence d'un agent déshydratant, pour obtenir le composé de formule XXV :

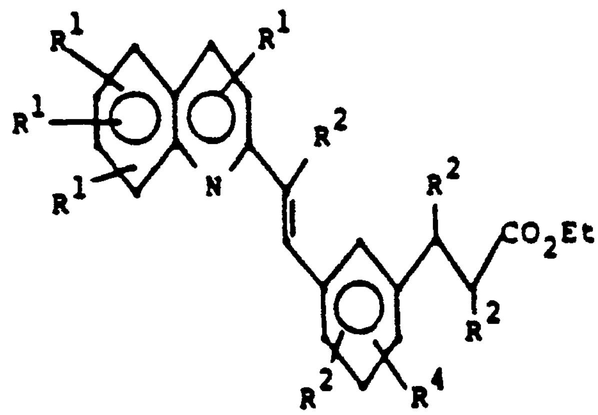

XXV

et, éventuellement, hydrolyser le composé de formule XXV pour obtenir le composé de formule XXVI :

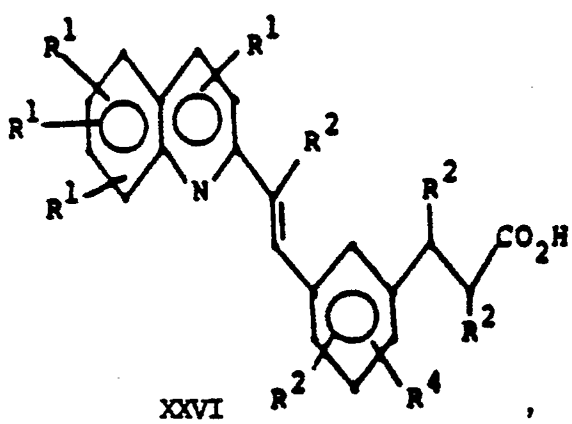

XXVI ,

dans laquelle $R^1$, $R^2$ et $R^4$ sont tels que définis dans la revendication 1.

7. Procédé selon l'une quelconque des revendications 1 à 6, appliqué à la préparation d'un composé de formule Ib :

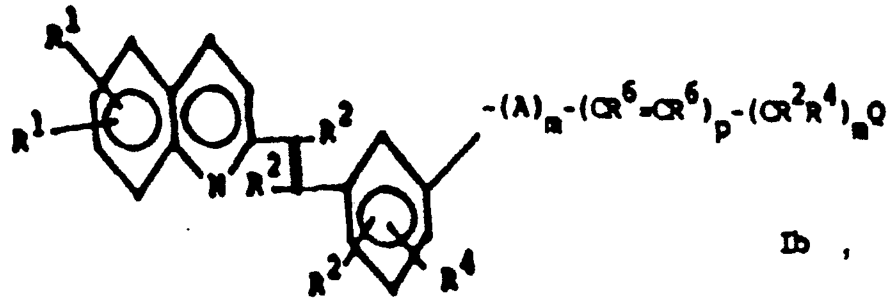

Ib ,

dans laquelle $R^1$ est H, un halogène, $CH_3$, $-CF_3$ ou $SCF_3$; $R^2$ est H, un groupe alkyle en $C_1$-$C_3$, alcényle en $C_2$-$C_3$ ou $-CF_3$; et $R^4$, $R^6$, m, p et A sont tels que définis dans la revendication 1; et de composés qui sont ses sels pharmaceutiquement acceptables.

**8.** Procédé selon l'une quelconque des revendications 1 à 6, appliqué à la préparation d'un composé de formule Ic :

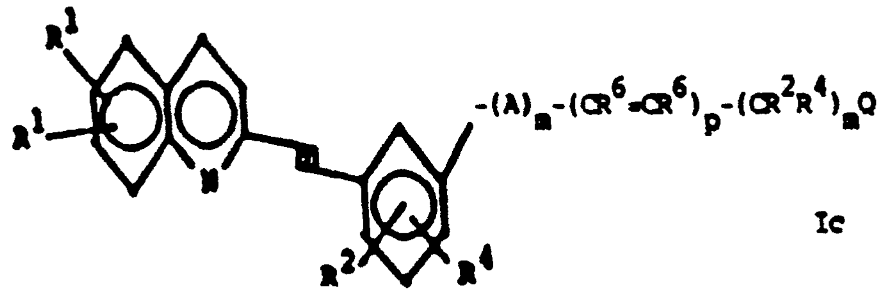

Ic ,

dans laquelle les variables sont telles que définies dans la revendication 7, et de composés qui sont ses sels pharmaceutiquement acceptables.

**9.** Procédé selon l'une quelconque des revendications 1 à 6, appliqué à la préparation des composés suivants tels que définis dans la revendication 1 :
ester méthylique de l'acide 4-(2-(quinoléine-2-yl)éthényl)benzoïque;
acide 4-(2-(quinoléine-2-yl)éthényl)benzoïque;
ester éthylique de l'acide 3-(2-(quinoléine-2-yl)éthényl)benzènepropanoïque;
acide 3-(2-(quinoléine-2-yl)éthényl)benzènepropanoïque;
ester méthylique de l'acide 3-(2-(quinoléine-2-yl)éthényl)-γ-oxo-β-méthylbenzènebutanoïque;
ester méthylique de l'acide 3-(2-(quinoléine-2-yl)éthényl)-γ-hydroxy-β-méthylbenzènebutanoïque;
lactone de l'acide [βR,γS et βS,γR]-3-(2-quinoléine-2-yl)éthényl)-γ-hydroxy-β-méthylbenzènebutanoïque;
lactone de l'acide [βS,γS et βR,γR]-3-(2-quinoléine-2-yl)éthényl)-γ-hydroxy-β-méthylbenzènebutanoïque;
sel de sodium de l'acide [βR,γS et βS,γR]-3-(2-(quinoléine-2-yl)éthényl)-γ-hydroxy-β-méthylbenzènebutanoïque;
sel de sodium de l'acide [βS,γS et βR,γR]-3-(2-(quinoléine-2-yl)éthényl)-γ-hydroxy-β-méthylbenzènebutanoïque;
acide 3-(2-(7-chloroquinoléine-2-yl)éthényl)-β-méthylbenzènepropanoïque;

**10.** Procédé de préparation d'une composition pharmaceutique utile pour antagoniser l'action des leucotriènes chez les mammifères, comprenant le mélange d'un composé préparé selon l'une quelconque des revendications 1 à 9, en une quantité efficace comme antagoniste des leucotriènes avec un véhicule pharmaceutiquement acceptable.

**11.** Procédé de préparation d'une composition pharmaceutique selon la revendication 10, comprenant en outre le mélange d'une quantité efficace d'un second ingrédient actif qui est un anti-inflammatoire non stéroïdien, un agent analgésique périphérique, un inhibiteur de cyclooxygénase, un antagoniste des

leucotriènes, un inhibiteur des leucotriènes, un antagoniste des récepteurs $H_2$, un agent antihistaminique, un antagoniste de la prostaglandine ou un antagoniste du thromboxane.

**12.** Utilisation d'un composé préparé selon l'une quelconque des revendications 1 à 9, pour préparer une composition utilisable pour empêcher la synthèse, l'action ou la libération de la SRS-A ou du leucotriène $D_4$ chez les mammifères.

**13.** Utilisation d'un composé préparé selon l'une quelconque des revendications 1 à 9, pour préparer une composition utilisable pour traiter les maladies inflammatoires de l'oeil des mammifères.

## Patentansprüche

**Patentansprüche für folgende Vertragsstaaten : BE, CH, DE, FR, GB, IT, LI, LU, NL, SE**

**1.** Verbindung der Formel Ia:

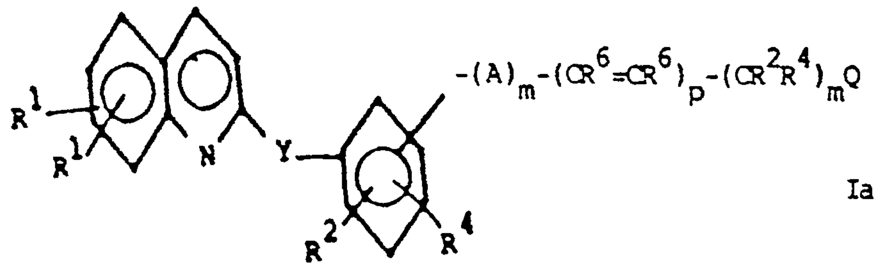

Ia

worin

| | |
|---|---|
| Y | $-(CR^2=CR^2)_n-$ oder $-(C\equiv C)_n-$ ist; |
| $R^1$ | H, Halogen, $C_{1-8}$-Alkyl, $C_{2-8}$-Alkenyl, $C_{2-8}$-Alkinyl, $-CF_3$, $-OR^2$, $-SR^2$, $-SOR^2$, $-SO_2R^2$, $-NR^2R^2$, -CHO, $-COOR^2$ , $-(C=O)R^2$, $-C(OH)R^2R^2$, -CN, $-NO_2$, substituiertes oder unsubstituiertes Phenyl, substituiertes oder unsubstituiertes Benzyl oder substituiertes oder unsubstituiertes Phenethyl ist; |
| $R^2$ | H, $C_{1-8}$-Alkyl, $C_{2-8}$-Alkenyl, $C_{2-8}$-Alkinyl, $-CF_3$, substituiertes oder unsubstituiertes Phenyl, substituiertes oder unsubstituiertes Benzyl oder substituiertes oder unsubstituiertes Phenethyl ist; |
| $R^4$ | H, Halogen, $-NO_2$, -CN, $-OR^2$, $-SR_2$ $-NR^2R^2$ oder $C_{1-8}$-Alkyl ist; |
| m | 0 oder eine 8 nicht übersteigende ganze Zahl ist; |
| n | 1 oder 2 ist; |
| p | 0 oder 1 ist; |
| A | $-CR^4R^4-$ oder $=C=O$ ist; |
| Q | $-COOR^2$, Tetrazol,<br>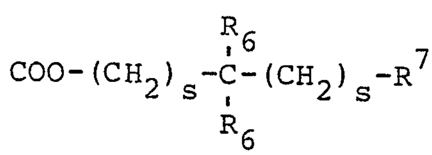<br>(worin s 0 oder eine 3 nicht übersteigende ganze Zahl ist, $R^6$ Wasserstoff oder $C_{1-4}$-Alkyl ist und $R^7$ $OR^8$, $SR^8$ oder $NHR^8$ ist, worin $R^8$ ein $C_{1-21}$-Kohlenwasserstoffrest oder ein $C_{1-21}$-Acylrest einer organischen acyclischen oder monocyclischen Carbonsäure mit nicht mehr als einem Heteroatom im Ring ist, oder $R^7$ ein monocyclisher oder bicyclischer heterocyclischer Rest mit 3 bis 12 nuklearen Kohlenstoffen und 1 oder 2 N-, S- und/oder O-Heteroatomen ist, wobei jeder Ring 5 oder 6 Atome aufweist), $-CONHSO_2R^{11}$, $-CONR^{10}R^{10}$ oder (jedoch nur, wenn die Summe von m, m und p größer als 0 ist) $-NHSO_3R^{11}$ ist; oder falls Q COOH ist und $R^3$ ein $R^4$ enthält, das -OH ist, dann Q und $R^4$ und die Kohlenstoffe, über die sie gebunden sind, unter Verlust von Wasser einen Lactonring bilden können; |
| $R^{10}$ | H, $C_{1-6}$-Alkyl, $-(C=O)R^{11}$, unsubstituiertes Phenyl oder unsubstituiertes Benzyl ist; und |
| $R^{11}$ | H, $C_{1-8}$-Alkyl, $C_{2-8}$-Alkenyl, $C_{2-8}$-Alkinyl, $-CF_3$ oder unsubstituiertes Phenyl, Benzyl oder |

Phenethyl ist;
wobei substituiertes Phenyl, Benzyl oder Phenethyl Phenyl, Benzyl oder Phenethyl mit einem oder zwei Substituenten am Benzolring, ausgewählt aus $C_{1-6}$-Alkyl, $NO_2$, $SCF_3$, Halogen, $COR^9$ CN und $CF_3$ bedeutet, worin $R^9$ $-OR^{10}$, $-SR^{10}$ oder $-NR^{10}R^{10}$ ist;
mit der Maßgabe, daß wenn zwei oder mehrere einer Variablen vorhanden sind, jede gleich oder verschieden von der/den anderen ist;
oder pharmazeutisch annehmbare Salze davon.

2. Verbindung der Formel Ib:

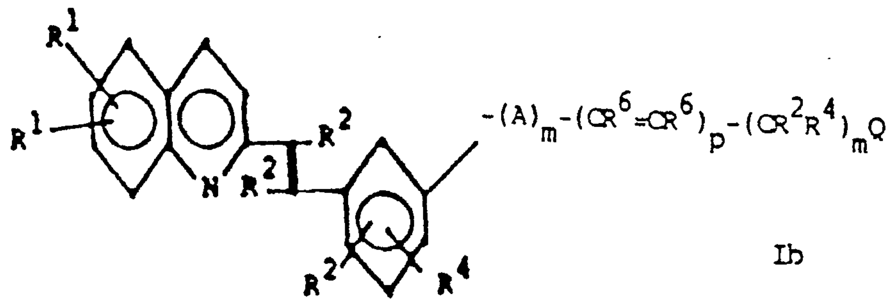

Ib

worin $R^1$ H, Halogen, $CH_3$, $-CF_3$ oder $SCF_3$ ist, $R^2$ H, $C_{1-3}$-Alkyl, $C_{2-3}$-Alkenyl oder $-CF_3$ ist und $R^4$, $R^6$, m, p und A wie in Anspruch 1 definiert sind, sowie Verbindungen, die pharmazeutisch annehmbare Salze davon sind.

3. Verbindung der Formel Ic:

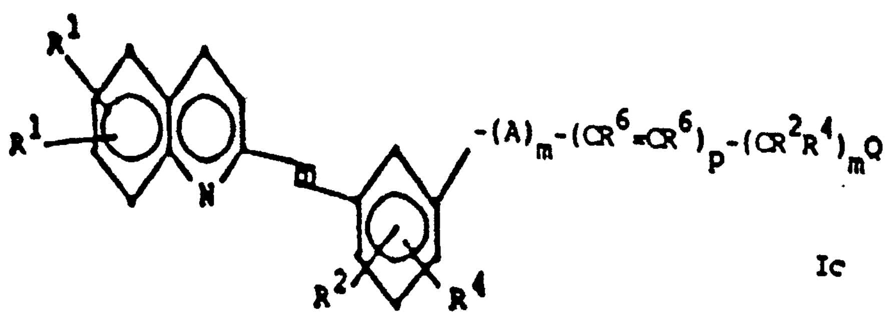

Ic

worin die Variablen wie in Anspruch 2 definiert sind, sowie Verbindungen, die pharmazeutisch annehmbare Salze davon sind.

4. Nachstehende Verbindungen nach Anspruch 1:
4-(2-(Chinolin-2-yl)ethenyl)benzoesäuremethylester;
4-(2-(Chinolin-2-yl)ethenyl)benzoesäure;
3-(2-(Chinolin-2-yl)ethenyl)benzolpropionsäureethylester;
3-(2-(Chinolin-2-yl)ethenyl)benzolpropionsäure;
3-(2-(Chinolin-2-yl)ethenyl)-γ-oxo-β-methylbenzolbuttersäuremethylester;
3-(2-(Chinolin-2-yl)ethenyl)-γ-hydroxy-β-methylbenzolbuttersäuremethylester;
βR,γS- und βS,γR-3-(2-Chinolin-2-yl)ethenyl)- -hydroxy-β-methylbenzolbuttersäurelacton;
βS, S- und βR, R-3-(2-Chinolin-2-yl)ethenyl)-γ-hydroxy-β-methylbenzolbuttersäurelacton;
βR,γS- und βS,γR-3-(2-(Chinolin-2-yl)ethenyl)-γ-hydroxy-β-methylbenzolbuttersäure,natriumsalz;
βS,γS- und βR,γR-3-(2-(Chinolin-2-yl)ethenyl)-γ-hydroxy-β-methylbenzolbuttersäure,natriumsalz;
3-(2-(7-Chlorchinolin-2-yl)ethenyl)-β-methylbenzolpropionsäure;

5. Zur Antagonisierung der Leukotrienwirkung in Säugetieren geeignete pharmazeutische Zusammensetzung, welche eine Verbindung nach einem der Ansprüche 1 bis 4 in einer als Leukotrienantagonist

wirksame Menge sowie einen pharmazeutisch annehmbaren Träger umfaßt.

6. Zusammensetzung nach Anspruch 5, welche weiterhin eine wirksame Menge eines zweiten wirksamen Bestandteils umfaßt, der ein nicht steroides entzündungshemmendes Mittel, ein peripheres Analgetikum, ein Cyclooxygenaseinhibitor, ein Leukotrienantagonist, ein Leukotrieninhibitor, ein $H_2$-Rezeptorantagonist, ein Antihistaminikum, ein Prostaglandinantagonist oder ein Thromboxanantagonist ist.

7. Verbindung nach einem der Ansprüche 1 bis 4 zur Verwendung bei der Vorbeugung der Synthese, Wirkung oder Freisetzung von SRS-A oder Leukotrien-$D_4$ in Säugetieren.

8. Verbindung nach einem der Ansprüche 1 bis 4 zur Behandlung von Entzündungskrankheiten des Auges in Säugetieren.

**Patentansprüche für folgenden Vertragsstaat : AT**

1. Verfahren zur Herstellung einer neuen Verbindung der Formel Ia:

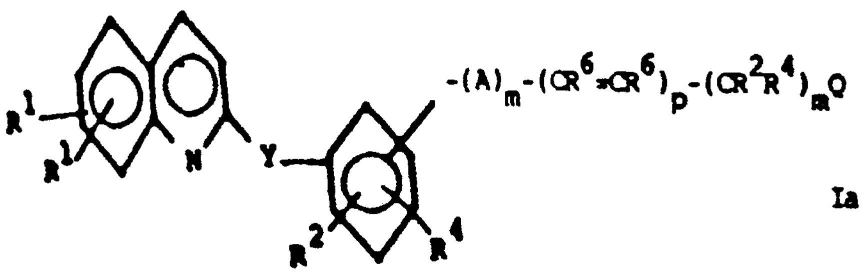

Ia ,

worin:

Y für $-(CR^2=CR^2)_n-$ oder $-(C\equiv C)_n-$ steht;

$R^1$ H, Halogen, $C_{1-8}$-Alkyl, $C_{2-8}$-Alkenyl, $C_{2-8}$-Alkinyl, $-CF_3$, $-OR^2$, $-SR^2$, $-SOR^2$, $-NR^2R^2$, -CHO, $-COOR^2$, $-(C=O)R^2$, $-C(OH)R^2R^2$, -CN, $-NO_2$, substituiertes oder unsubstituiertes Phenyl, substituiertes oder unsubstituiertes Benzyl oder substituiertes oder unsubstituiertes Phenethyl darstellt;

$R^2$ H, $C_{1-8}$-Alkyl, $C_{2-8}$-Alkenyl, $C_{2-8}$-Alkinyl, $-CF_3$, substituiertes oder unsubstituiertes Phenyl, substituiertes oder unsubstituiertes Benzyl oder substituiertes oder unsubstituiertes Phenethyl bedeutet;

$R^4$ für H, Halogen, $-NO_2$, -CN, $-OR^2$, $-SR^2$, $-NR^2R^2$ oder $C_{1-8}$-Alkyl steht;

m 0 oder eine ganze Zahl ist, welche 8 nicht überschreitet;

n den Wert 1 oder 2 besitzt;

p den Wert 0 oder 1 besitzt;

A $-CR^4R^4-$ oder $=C=O$ darstellt;

Q $-COOR^2$, Tetrazol,

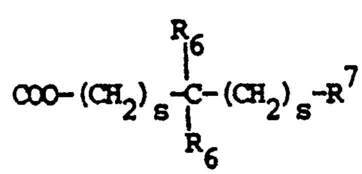

(worin s 0 oder eine ganze Zahl bedeutet, welche 3 nicht überschreitet, $R^6$ für Wasserstoff oder $C_{1-4}$-Alkyl steht, und $R^7$ $OR^8$, $SR^8$ oder $NHR^8$ darstellt, worin $R^8$ einen $C_{1-21}$-Kohlenwasserstoffrest oder einen $C_{1-21}$-Acylrest einer organischen acyclischen oder monocyclischen Carbonsäure mit nicht mehr als einem Heteroatom im Ring bedeutet, oder $R^7$ einen monocyclischen oder bicyclischen heterocyclischen Rest mit 3 bis 12 Kohlenstoffatomen im Kern und 1 oder 2 N-, S- und/oder O-Heteroatomen darstellt, wobei jeder Ring 5 oder 6 Atome aufweist), $-CONHSO_2R^{11}$, $-CONR^{10}R^{10}$ oder (aber nur, wenn die Summe von m, n und p größer als O ist) $-NHSO_2R^{11}$ bedeutet; oder worin im Fall, daß Q für COOH

steht und $R^3$ einen Substituenten $R^4$ enthält, welcher -OH ist, Q und $R^4$ und die Kohlenstoffatome, durch welche sie gebunden sind, unter Abspaltung von Wasser einen Lactonring ausbilden können;

$R^{10}$ H, $C_{1-6}$-Alkyl, -(C=O)$R^{11}$, unsubstituiertes Phenyl oder unsubstituiertes Benzyl darstellt; und

$R^{11}$ H, $C_{1-8}$-Alkyl, $C_{2-8}$-Alkenyl, $C_{2-8}$-Alkinyl, $-CF_3$ oder unsubstituiertes Phenyl, Benzyl oder Phenethyl darstellt;

worin "substituiertes Phenyl, Benzyl oder Phenethyl" Phenyl, Benzyl oder Phenethyl mit einem oder zwei Substituenten am Benzolring bedeutet, welche Substituenten unter $C_{1-6}$-Alkyl, $NO_2$, $SCF_3$, Halogen, $COR^9$, CN und $CF_3$, worin $R^9$ für $-OR^{10}$, $-SR^{10}$ oder $-NR^{10}R^{10}$ steht, ausgewählt sind;
mit der Maßgabe, daß für jede beliebige Variable bei Vorliegen von zwei oder mehreren Variablen jede Variable die gleiche oder eine unterschiedliche Bedeutung wie die andere (die anderen) besitzen kann;
oder der pharmazeutisch annehmbaren Salze hievon,
umfassend:

Erhitzen eines Anilinderivats der Formel II:

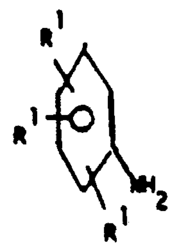

II

mit einem Crotonaldehyd in Gegenwart einer starken Mineralsäure, um das substituierte Chinaldinderivat der Formel III:

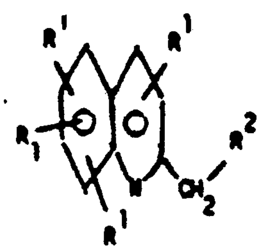

III

zu erhalten;

Abtrennen der gegebenenfalls erhaltenen Regioisomere des Chinaldinderivats der Formel III;

Behandeln des Derivats der Formel III mit einer Halogenquelle in Gegenwart von Licht, um das Chinaldin der Formel IV:

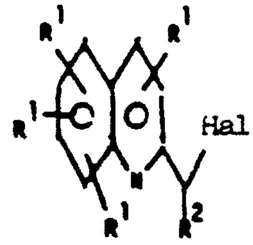

IV

zu erhalten;

Behandeln des Chinaldins der Formel IV mit Triphenylphosphin in einem inerten Lösungsmittel, um das Phosphoniumsalz der Formel V:

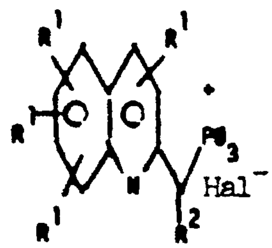

V

zu erhalten;

Umsetzen des Chinaldinderivats der Formel V mit einer starken Base in einem inerten Lösungsmittel bei niedriger Temperatur;

Umsetzen des entstehenden Ylids mit dem Dialdehyd der Formel VI:

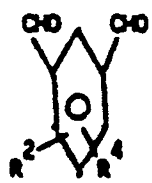

VI

um das Vinylchinolin der Formel VII:

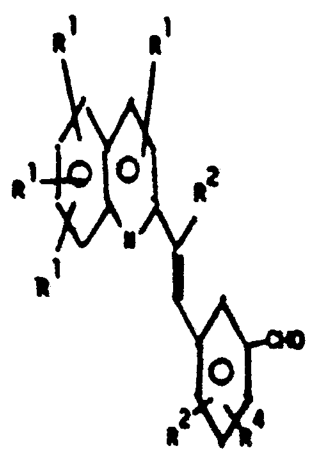

VII

zu erhalten;

Umsetzen des Chinolins der Formel VII mit einem Ylid der Formel VIII:

$\phi_3P=CH\text{-}(CH_2)_{(m-2)}\text{-}Q$ VIII,

um das Addukt der Formel IX:

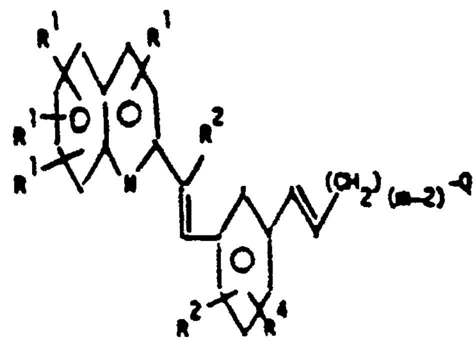

IX

zu erhalten,

und gegebenenfalls Behandeln des Adduktes der Formel IX mit Wasserstoff in Gegenwart eines geeigneten Katalysators, um ein 2-Styrylchinolinderivat der Formel X:

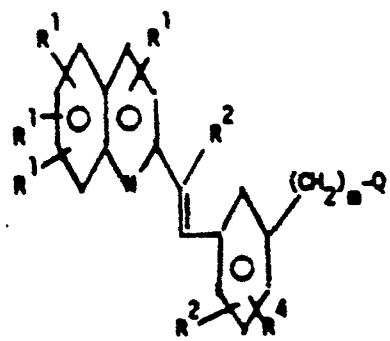

X

zu erhalten; worin $R^1$, $R^2$, $R^4$, Q und m wie vorstehend definiert sind.

2. Verfahren nach Anspruch 1, worin das Dialdehyd der Formel VI mit dem Ylid der Formel VIII umgesetzt wird und nach Reduzieren der Doppelbindung die so erhaltene Verbindung mit dem Chinaldinderivat der Formel V umgesetzt wird, um das 2-Styrylchinolinderivat der Formel X zu erhalten.

3. Verfahren zur Herstellung einer Verbindung nach Anspruch 1, umfassend:

Umsetzen des Aldehyds der Formel VII:

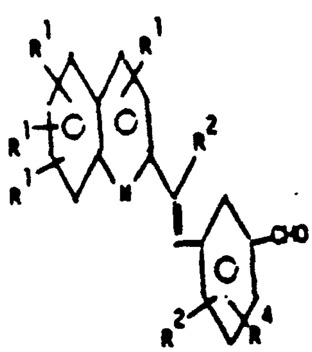

VII

mit einem Grignard-Reagens der Formel XI:

$R^2CH_2MgHal$ XI

oder einem äquivalenten Organolithiumreagens, um den Alkohol der Formel XII:

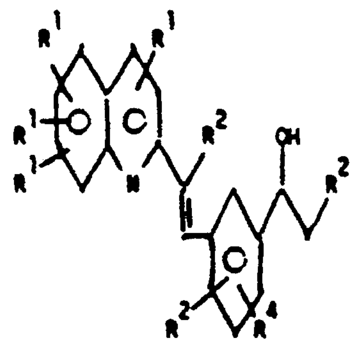

XII

zu erhalten;

Oxidieren des Alkohols der Formel XII mit einem geeigneten Oxidationsmittel, um das Keton der Formel XIII:

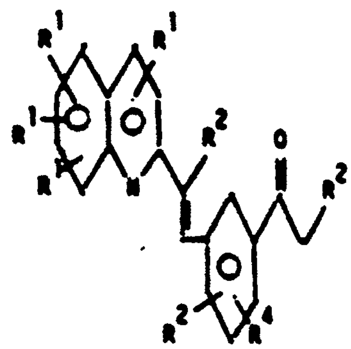

XIII

zu erhalten;

Deprotonieren des Ketons der Formel XIII mit einer starken Base in einem inerten Lösungsmittel bei niedriger Temperatur;

Umsetzen des so erhaltenen Enolats mit einer Alkansäure, einem Ester, einem Nitril oder einem Tetrazol, welche endständig mit einer Leaving-Gruppe substituiert sind, um das Addukt der Formel XIV:

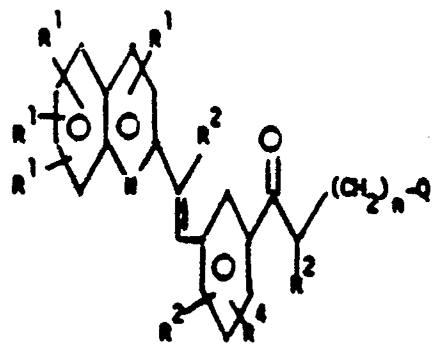

XIV

zu erhalten;

und gegebenenfalls Reduzieren des Adduktes der Formel XIV mit einem geeigneten Reduktionsmittel, um den Chinolinalkohol der Formel XV:

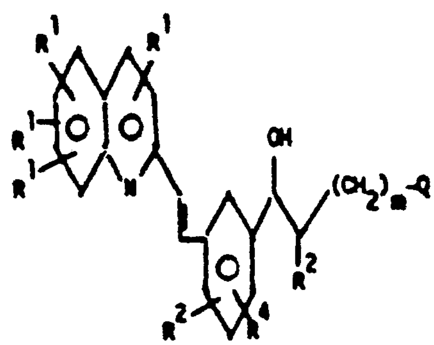

XV

zu erhalten; worin $R^1$, $R^2$, $R^4$, Q und m wie in Anspruch 1 definiert sind.

4. Verfahren zur Herstellung einer Verbindung nach Anspruch 1, umfassend:
Erhitzen eines Chinolinderivats der Formel III:

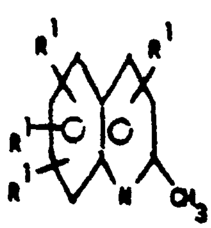

III

mit einem Dialdehyd der Formel VI:

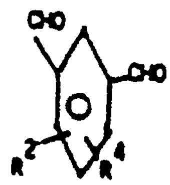

VI

in Gegenwart eines Dehydratisierungsmittels, um einen 2-Styrylchinolinaldehyd der Formel VII:

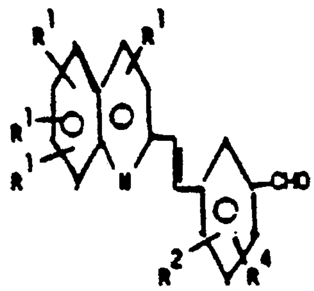

VII

zu erhalten;

und Behandeln des 2-Styrylchinolinaldehyds der Formel VII gemäß einem der Ansprüche 1 bis 3, um die entsprechenden Verbindungen, worin $R^1$, $R^2$, $R^4$, Q und m wie in Anspruch 1 definiert sind, zu erhalten.

**5.** Verfahren zur Herstellung einer Verbindung nach Anspruch 1, umfassend:

Kondensieren eines Dialdehyds der Formel VI:

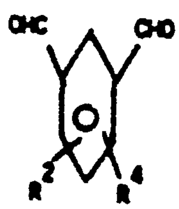

VI

mit einem Ylid der Formel VIII:

$\phi_3P=CH\text{-}(CH_2)_{m\text{-}2}\text{-}Q$ VIII,

um einem olefinischen Aldehyd der Formel XVI:

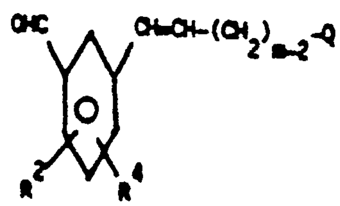

XVI

zu erhalten;

Reduzieren des olefinischen Aldehyds der Formel XVI mit Wasserstoff in Gegenwart eines geeigneten Katalysators, um den Aldehyd der Formel XVII:

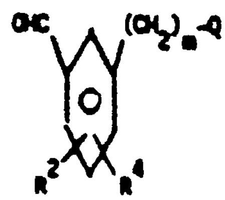

XVII

zu erhalten,

und Kondensieren des Aldehyds der Formel XVII mit einem Chinaldin der Formel III:

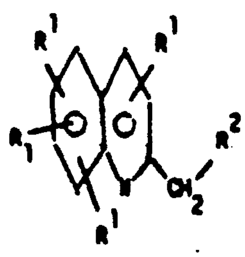

III

in Gegenwart eines Dehydratisierungsmittels, um eine Verbindung der Formel X:

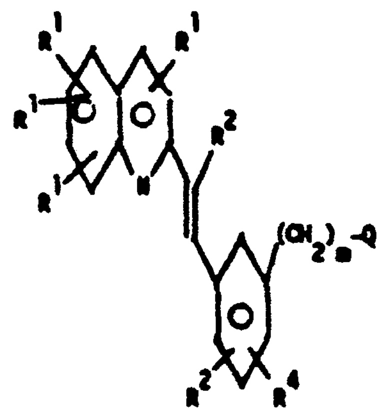

X

zu erhalten; worin $R^1$, $R^2$, $R^4$, Q und m wie in Anspruch 1 definiert sind.

**6.** Verfahren zur Herstellung einer Verbindung nach Anspruch 1, umfassend

Zur-Hälfte-Reduzieren und Schützen der so gebildeten Alkoholgruppe eines Dialdehyds der Formel VI:

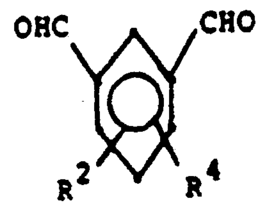

VI,

um eine Verbindung der Formel XVIII:

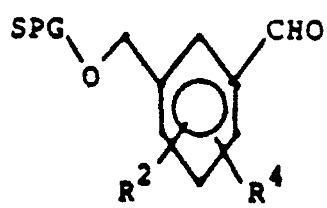

XVIII,

worin SPG eine Silylschutzgruppe darstellt, zu erhalten;

Umsetzen der Verbindung der Formel XVIII mit einer Verbindung der Formel XIXa:

$R^2$-Li XIXa,

worin $R^2$ eine andere Bedeutung als Wasserstoff besitzt, um eine Verbindung der Formel XX:

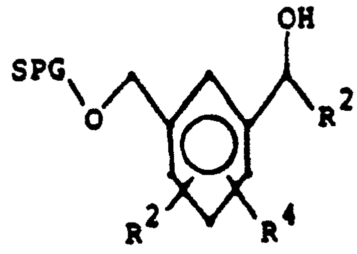

XX

zu erhalten;

Oxidieren der Verbindung der Formel XX mit einem geeigneten Oxidationsmittel, um eine Verbindung der Formel XXI:

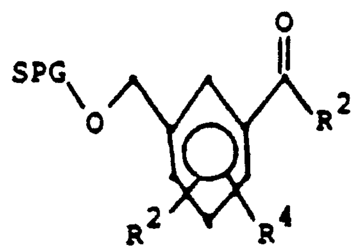

XXI

zu erhalten;

Kondensieren der Verbindung der Formel XXI mit dem Silylester der Formel XXII:

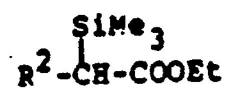

XXII;

Reduzieren des so erhaltenen $\alpha,\beta$-ungesättigten Esters zu der Verbindung der Formel XXIII:

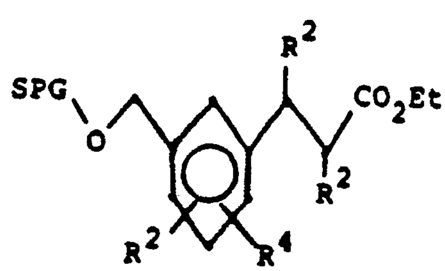

XXIII;

Überführen der geschützten Alkoholgruppe der Verbindung der Formel XXIII durch bekannte Vorgangsweise in eine Aldehydgruppe, um den Aldehyd der Formel XXIV:

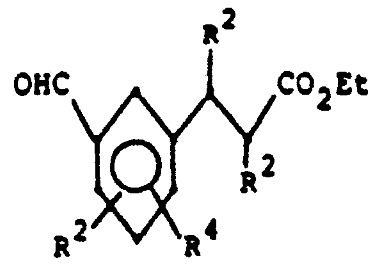

XXIV

zu erhalten;

Kondensieren des Aldehyds der Formel XXIV mit einer Verbindung der Formel III:

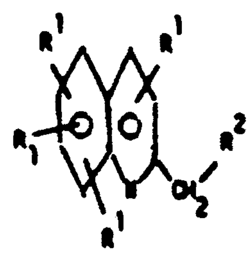

III

in Gegenwart eines Dehydratisierungsmittels, um die Verbindung der Formel XXV:

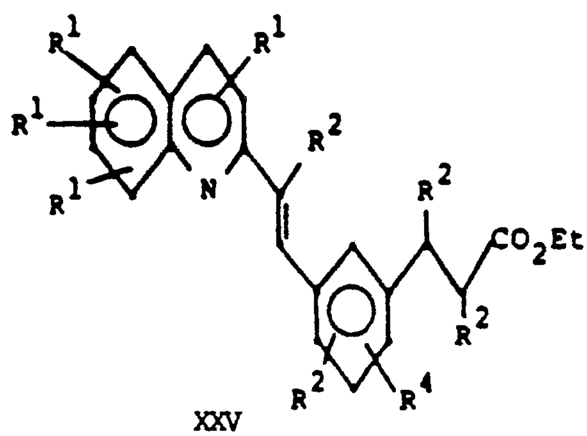

XXV

zu erhalten,

und gegebenenfalls Hydrolysieren der Verbindung der Formel XXV, um die Verbindung der Formel XXVI:

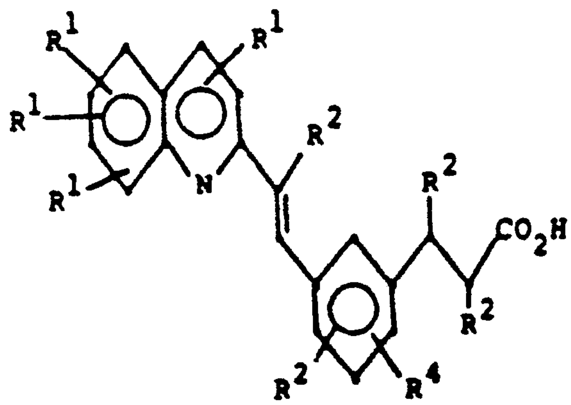

XXVI

zu erhalten; worin $R^1$, $R^2$ und $R^4$ wie in Anspruch 1 definiert sind.

7. Verfahren nach einem der Ansprüche 1 bis 6, angewandt auf die Herstellung einer Verbindung der Formel Ib:

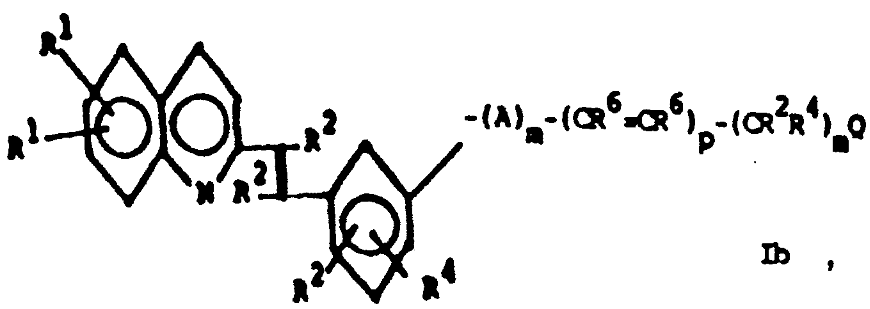

Ib ,

worin $R^1$ für H, Halogen, $CH_3$, $-CF_3$ oder $SCF_3$ steht; $R^2$ H $C_{1-3}$-Alkyl, $C_{2-3}$-Alkenyl oder $-CF_3$ bedeutet und $R^4$, $R^6$, m, p und A wie in Anspruch 1 definiert sind; und von Verbindungen, welche pharmazeutisch annehmbare Salze hievon sind.

8. Verfahren nach einem der Ansprüche 1 bis 6, angewandt auf die Herstellung einer Verbindung der Formel 1c:

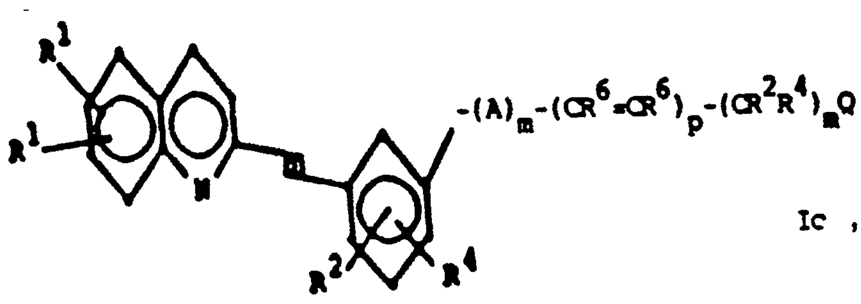

Ic ,

worin die Variablen wie in Anspruch 7 definiert sind, und von Verbindungen, welche pharmazeutisch annehmbare Salze hievon sind.

9. Verfahren nach einem der Ansprüche 1 bis 6, angewandt auf die Herstellung von einer der Verbindungen nach Anspruch 1:
4-(2-(Chinolin-2-yl)ethenyl)benzoesäuremethylester;
4-(2-(Chinolin-2-yl)ethenyl)benzoesäure;
3-(2-(Chinolin-2-yl)ethenyl)benzolpropansäureethylester;

3-(2-(Chinolin-2-yl)ethenyl)benzolpropansäure;
3-(2-(Chinolin-2-yl)ethenyl)-γ-oxo-β-methylbenzolbutansäuremethylester;
3-(2-(Chinolin-2-yl)ethenyl)-γ-hydroxy-β-methylbenzolbutansäuremethylester;
[βR, γS und βS, γR]-3-(2-Chinolin-2-yl)ethenyl)-γ-hydroxy-β-methylbenzolbutansäurelacton;
[βS, γS und βR, γR]-3-(2-Chinolin-2-yl)ethenyl-γ-hydroxy-β-methylbenzolbutansäurelacton;
[βR, γS und βS, γR]-3-(2-Chinolin-2-yl)ethenyl)-γ-hydroxy-β-methylbenzolbutansäure-natriumsalz;
[βS, γS und βR, γR]-3-(2-Chinolin-2-yl)ethenyl)-γ-hydroxy-β-methylbenzolbutansäure-natriumsalz;
3-(2-(7-Chlorchinolin-2-yl)ethenyl)-β-methylbenzolpropansäure;

**10.** Verfahren zur Herstellung einer pharmazeutischen Zusammensetzung, welche zum Antagonisieren von Leukotrienwirkung bei Säugetieren nützlich ist, umfassend das Vermischen einer nach einem der Ansprüche 1 bis 9 hergestellten Verbindung in einer derartigen Menge, daß sie als Leukotrienantagonist wirksam ist, mit einem pharmazeutisch annehmbaren Träger.

**11.** Verfahren zur Herstellung einer pharmazeutischen Zusammensetzung nach Anspruch 10, welche zusätzlich das Vermischen mit einer wirksamen Menge eines zweiten wirksamen Bestandteils umfaßt, welcher ein nicht-steroides, entzündungshemmendes Arzneimittel; ein peripheres Analgetikum; ein Cyclooxygenaseinhibitor; ein Leukotrienantagonist; ein Leukotrieninhibitor; ein $H_2$-Rezeptor-Antagonist; ein Antihistaminikum; ein Prostaglandin-Antagonist oder ein Thromboxan-Antagonist ist.

**12.** Verwendung einer nach einem der Ansprüche 1 bis 9 hergestellten Verbindung zur Herstellung einer Zusammensetzung, welche zur Verhinderung der Synthese, der Wirkung oder der Freisetzung von SRS-A und Leukotrien $D_4$ bei Säugetieren verwendet wird.

**13.** Verwendung einer nach einem der Ansprüche 1 bis 9 hergestellten Verbindung zur Herstellung einer Zusammensetzung, welche zur Behandlung entzündlicher Leiden des Auges bei Säugetieren angewandt wird.